# EUROPEAN PATENT SPECIFICATION

(11) **EP 4 561 454 B1**
(45) Date of publication and mention of the grant of the patent: **09.12.2026**
(21) Application number: 23847237.7
(22) Date of filing: 25.07.2023
(51) Int. Cl.: G06T 7/73, A61B 8/12, A61B 90/90, G09B 23/00, A61M 5/00, A61B 5/00, A61M 5/14, A61M 39/10

(54) **SYSTEM AND METHOD FOR VASCULAR ACCESS MANAGEMENT**
SYSTEM UND VERFAHREN ZUR VERWALTUNG DES VASKULÄREN ZUGANGS
SYSTÈME ET PROCÉDÉ DE GESTION D'ACCÈS VASCULAIRE

(30) Priority: 26.07.2022 IN 202211042859
(43) Date of publication of application: 04.06.2025
(73) Proprietor: Becton, Dickinson and Company, Franklin Lakes, NJ 07417 (US)
(72) Inventor: SEYED VAHEDEIN, Yashar, Mountain View, California 94040 (US); GUNTHER, Matthew, J., Boulder, Colorado 80305 (US); S DEYAGOND, Sagar, Karnataka Vijayapura 586 109 (IN); WITT, Erik, Kurt, Oakland, New Jersey 07436 (US); DEBOER, Dylan, Gregory, Vernon Hills, Illinois 60061 (US); TRAINA, Zachary, Jarrod, Denville, New Jersey 07834 (US); MCKINNON, Austin, J., Herriman, Utah 84096 (US)
(74) Representative: dompatent
(86) International application number: PCT/US2023/028521
(87) International publication number: WO 2024/025850

(56) References cited:
- AU-A1- 2020 219 832
- US-A1- 2011 306 376
- US-A1- 2012 242 851
- US-A1- 2015 209 113
- US-A1- 2015 209 113
- US-A1- 2017 019 657
- US-A1- 2019 076 200
- US-A1- 2019 180 467
- US-A1- 2019 329 407
- US-A1- 2020 159 313

## Description

### CROSS-REFERENCE TO RELATED APPLICATION

The present application claims priority to Indian Provisional Patent Application No. 202211042859, entitled "System and Method for Vascular Access Management", filed July 26, 2022, the entire disclosure of which is hereby incorporated by reference in its entirety.

### BACKGROUND

When a patient is admitted to a hospital, a variety of disposable drug delivery devices (e.g., a needleless connector, an IV tubing, an extension set, a catheter, etc.) may be attached to the patient to deliver medication from an infusion pump to the patient at one or more catheter insertion sites. A single IV line may be constructed from multiple devices. A catheter with multiple lumens may form parts of multiple IV lines, which may be referred to as a "catheter tree". Nurses may label IV lines with color stickers (or other methods) to track medication infused from each individual pump module of the infusion pump to each IV line.

Multiple IV lines attached to a patient may have implications on drug compatibility for the patient. Further, during a patient stay, the components of the IV lines may be replaced and/or the line configurations may be changed to accommodate different patient care needs. Nurses may need to keep track of these changes to the IV lines by documenting a dwell time of components and/or connections thereof to ensure line cleanliness and drug compatibility. Document US20150209113A1 uses a wearable device to capture procedure images and present guidance in the clinician's field of view. The vascular access embodiment locates a suitable vein using ultrasound and camera images, then displays a virtual vein trace for needle placement. It verifies tagged vascular access items and records insertion details in a patient data system.

### SUMMARY

Accordingly, provided are improved systems, devices, products, apparatus, and/or methods for vascular access management. The invention is defined by the appended claims.

According to some non-limiting embodiments or aspects, provided is a system, comprising: at least one processor programmed and/or configured to: obtain an image of a plurality of medical devices, captured by an image capture device; determine, based on the image, position information associated with a three-dimensional (3D) position of the plurality of medical devices relative to the image capture device; determine, based on the position information, pairs of medical devices of the plurality of medical devices that are connected to each other; and generate, based on the pairs of medical devices that are determined to be connected to each other, a representation of at least one IV line including the pairs of medical devices that are determined to be connected to each other.

In some non-limiting embodiments or aspects, the at least one processor is programmed and/or configured to determine the pairs of medical devices that are connected to each other by: for each pair of medical devices of the plurality of medical devices, determining, based on the position information associated with that pair of medical devices, a probability that that pair of medical devices are connected to each other.

In some non-limiting embodiments or aspects, the at least one processor is programmed and/or configured to determine the pairs of medical devices that are connected to each other by: for each pair of medical devices of the plurality of medical devices, determining, based on the position information associated with that pair of medical devices, the following parameters: a distance between center points associated with that pair of medical devices, an angular difference between orientations of that pair medical devices, and an angle from collinearity of that pair of medical devices, wherein the probability that that pair of medical devices is connected is determined based on the distance between the center points of that pair of medical devices, the angular difference between the orientations of that pair of medical devices, and the angle from collinearity of that pair of medical devices.

In some non-limiting embodiments or aspects, for each medical device of the plurality of medical devices, that medical device is determined to be connected to another medical device in a pair of medical devices including that medical device that is associated with a highest probability of the pairs of medical devices including that medical device.

In some non-limiting embodiments or aspects, the at least one processor is programmed and/or configured to determine the position information associated with the 3D position of the plurality of medical devices relative to the image capture device by: determining, based on the image, a type of each medical device of the plurality of medical devices, wherein, for a pair of medical devices of the plurality of medical devices, the probability that that pair of medical devices are connected to each other is further determined based on the type of each medical device in that pair of medical devices.

In some non-limiting embodiments or aspects, the at least one processor is programmed and/or configured to determine the pairs of medical devices that are connected to each other by: identifying, based on the type of each medical device of the plurality of medical devices, a set of medical devices of the plurality of medical devices that are associated with a preferred IV line architecture; and for a pair of medical devices included in the set of medical devices associated with the preferred IV line architecture, adjusting a weight used to determine whether the pair of medical devices are connected to each other.

In some non-limiting embodiments or aspects, the at least one processor is programmed and/or configured to determine the pairs of medical devices that are connected to each other by: determining, based on the type of each medical device of the plurality of medical devices, that no medical devices of the plurality of medical devices are associated with a preferred IV line architecture; and in response to determining that no medical devices of the plurality of medical devices are associated with the preferred IV line architecture, prompting a user to obtain another image.

In some non-limiting embodiments or aspects, a first group of medical devices of the plurality of medical devices is associated with a plurality of fiducial markers, wherein the plurality of fiducial markers encapsulates a plurality of identifiers associated with the first group of medical devices and pose information associated with the 3D position of the plurality of fiducial markers, and wherein the at least one processor is programmed and/or configured to determine the position information associated with the 3D position of the plurality of medical devices relative to the image capture device by: determining, based on the image, the plurality of identifiers associated with the first group of medical devices and the pose information associated with the 3D position of the plurality of fiducial markers, wherein, for each medical device of the first group of medical devices, the position information associated with the 3D position of that medical device relative to the image capture device is determined as the 3D position of a fiducial marker associated with that medical device relative to the image capture device.

In some non-limiting embodiments or aspects, the plurality of fiducial markers includes a plurality of AprilTags.

In some non-limiting embodiments or aspects, for each medical device of the first group of medical devices, the 3D position of the fiducial marker associated with that medical device relative to the image capture device includes x, y, and z coordinates of the fiducial marker and directional vectors for Z, Y, and X axes of the fiducial marker.

In some non-limiting embodiments or aspects, the plurality of identifiers is associated with a plurality of types of medical devices.

In some non-limiting embodiments or aspects, the plurality of identifiers includes a plurality of unique identifiers.

In some non-limiting embodiments or aspects, a second group of medical devices of the plurality of medical devices is not associated with a fiducial marker, and wherein the at least one processor is programmed and/or configured to determine the position information associated with the 3D position of the plurality of medical devices relative to the image capture device by: for each medical device of the second group of medical devices, determining, based on the image, using an object recognition technique, a type of that medical device and the 3D position of that medical device relative to the image capture device.

In some non-limiting embodiments or aspects, the at least one processor is programmed and/or configured to obtain the image of the plurality of medical devices further by: capturing, with the image capture device, the image of the plurality of medical devices.

In some non-limiting embodiments or aspects, the image includes a series of images.

In some non-limiting embodiments or aspects, the image capture device captures the series of images using a burst capture technique.

In some non-limiting embodiments or aspects, the image capture device includes a stereo camera, and wherein the position information associated with the 3D position of the plurality of medical devices relative to the image capture device is determined using a Structure from Motion (SfM) algorithm.

In some non-limiting embodiments or aspects, the image capture device includes a LiDAR system, and wherein the image includes a LiDAR point cloud.

In some non-limiting embodiments or aspects, the at least one processor is programmed and/or configured to generate the representation of the at least one IV line by automatically displaying, on the image, lines connecting the pairs of medical devices that are determined to be connected to each other in the at least on IV line.

In some non-limiting embodiments or aspects, the at least one IV line is associated with at least one pump of an infusion pump in the representation.

In some non-limiting embodiments or aspects, the plurality of fiducial markers is rigidly affixed to rigid portions of the first group of medical devices such that the plurality of fiducial markers cannot translate along and rotate around the first group of medical devices.

In some non-limiting embodiments or aspects, the at least one processor is programmed and/or configured to: determine, based on the pairs of medical devices that are determined to be connected to each other, a dwell time indicating a duration of time a medical device of the plurality of medical devices is connected to another medical device of the plurality of medical devices; and in response to the dwell time satisfying a threshold dwell time, provide, via the user device, an alert associated with the medical device. A dwell time may also indicate whether/if a tagged disposable has been changed since the last time the tagged disposable was scanned by the system, or whether a tagged disposable has been detected for a duration specified by a service provider or hospital.

According to some non-limiting embodiments or aspects, provided is a method, comprising: obtaining, with at least one processor, an image of a plurality of medical devices, captured by an image capture device; determining, with at least one processor, based on the image, position information associated with a three-dimensional (3D) position of the plurality of medical devices relative to the image capture device; determining, with the at least one processor, based on the position information, pairs of medical devices of the plurality of medical devices that are connected to each other; and generating, with the at least one processor, based on the pairs of medical devices that are determined to be connected to each other, a representation of at least one IV line including the pairs of medical devices that are determined to be connected to each other.

In some non-limiting embodiments or aspects, determining the pairs of medical devices that are connected to each other further includes: for each pair of medical devices of the plurality of medical devices, determining, based on the position information associated with that pair of medical devices, a probability that that pair of medical devices are connected to each other.

In some non-limiting embodiments or aspects, determining the pairs of medical devices that are connected to each other further includes: for each pair of medical devices of the plurality of medical devices, determining, based on the position information associated with that pair of medical devices, the following parameters: a distance between center points associated with that pair of medical devices, an angular difference between orientations of that pair medical devices, and an angle from collinearity of that pair of medical devices, wherein the probability that that pair of medical devices is connected is determined based on the distance between the center points of that pair of medical devices, the angular difference between the orientations of that pair of medical devices, and the angle from collinearity of that pair of medical devices.

In some non-limiting embodiments or aspects, for each medical device of the plurality of medical devices, that medical device is determined to be connected to another medical device in a pair of medical devices including that medical device that is associated with a highest probability of the pairs of medical devices including that medical device.

In some non-limiting embodiments or aspects, determining the position information associated with the 3D position of the plurality of medical devices relative to the image capture device further includes: determining, based on the image, a type of each medical device of the plurality of medical devices, wherein, for a pair of medical devices of the plurality of medical devices, the probability that that pair of medical devices are connected to each other is further determined based on the type of each medical device in that pair of medical devices.

In some non-limiting embodiments or aspects, determining the pairs of medical devices that are connected to each other further includes: identifying, based on the type of each medical device of the plurality of medical devices, a set of medical devices of the plurality of medical devices that are associated with a preferred IV line architecture; and for a pair of medical devices included in the set of medical devices associated with the preferred IV line architecture, adjusting a weight used to determine whether the pair of medical devices are connected to each other.

In some non-limiting embodiments or aspects, determining the pairs of medical devices that are connected to each other further includes: determining, based on the type of each medical device of the plurality of medical devices, that no medical devices of the plurality of medical devices are associated with a preferred IV line architecture; and in response to determining that no medical devices of the plurality of medical devices are associated with the preferred IV line architecture, prompting a user to obtain another image.

In some non-limiting embodiments or aspects, a first group of medical devices of the plurality of medical devices is associated with a plurality of fiducial markers, wherein the plurality of fiducial markers encapsulates a plurality of identifiers associated with the first group of medical devices and pose information associated with the 3D position of the plurality of fiducial markers, and wherein determining the position information associated with the 3D position of the plurality of medical devices relative to the image capture device includes: determining, based on the image, the plurality of identifiers associated with the first group of medical devices and the pose information associated with the 3D position of the plurality of fiducial markers, wherein, for each medical device of the first group of medical devices, the position information associated with the 3D position of that medical device relative to the image capture device is determined as the 3D position of a fiducial marker associated with that medical device relative to the image capture device.

In some non-limiting embodiments or aspects, the plurality of fiducial markers includes a plurality of AprilTags.

In some non-limiting embodiments or aspects, for each medical device of the first group of medical devices, the 3D position of the fiducial marker associated with that medical device relative to the image capture device includes x, y, and z coordinates of the fiducial marker and directional vectors for Z, Y, and X axes of the fiducial marker.

In some non-limiting embodiments or aspects, the plurality of identifiers is associated with a plurality of types of medical devices.

In some non-limiting embodiments or aspects, the plurality of identifiers includes a plurality of unique identifiers.

In some non-limiting embodiments or aspects, a second group of medical devices of the plurality of medical devices is not associated with a fiducial marker, and wherein determining the position information associated with the 3D position of the plurality of medical devices relative to the image capture device further includes: for each medical device of the second group of medical devices, determining, based on the image, using an object recognition technique, a type of that medical device and the 3D position of that medical device relative to the image capture device.

In some non-limiting embodiments or aspects, obtaining the image of the plurality of medical devices further includes: capturing, with the image capture device, the image of the plurality of medical devices.

In some non-limiting embodiments or aspects, the image includes a series of images.

In some non-limiting embodiments or aspects, the image capture device captures the series of images using a burst capture technique.

In some non-limiting embodiments or aspects, the image capture device includes a stereo camera, and wherein the position information associated with the 3D position of the plurality of medical devices relative to the image capture device is determined using a Structure from Motion (SfM) algorithm.

In some non-limiting embodiments or aspects, the image capture device includes a LiDAR system, and wherein the image includes a LiDAR point cloud.

In some non-limiting embodiments or aspects, generating the representation of the at least one IV line includes automatically displaying, on the image, lines connecting the pairs of medical devices that are determined to be connected to each other in the at least on IV line.

In some non-limiting embodiments or aspects, the at least one IV line is associated with at least one pump of an infusion pump in the representation.

In some non-limiting embodiments or aspects, the plurality of fiducial markers is rigidly affixed to rigid portions of the first group of medical devices such that the plurality of fiducial markers cannot translate along and rotate around the first group of medical devices.

In some non-limiting embodiments or aspects, the method further includes: determining, with the at least one processor, based on the pairs of medical devices that are determined to be connected to each other, a dwell time indicating a duration of time a medical device of the plurality of medical devices is connected to another medical device of the plurality of medical devices; and in response to the dwell time satisfying a threshold dwell time, providing, with the at least one processor, via the user device, an alert associated with the medical device.

Further non-limiting embodiments or aspects are set forth in the following numbered clauses:
Clause 1. A system, comprising: at least one processor programmed and/or configured to: obtain an image of a plurality of medical devices, captured by an image capture device; determine, based on the image, position information associated with a three-dimensional (3D) position of the plurality of medical devices relative to the image capture device; determine, based on the position information, pairs of medical devices of the plurality of medical devices that are connected to each other; and generate, based on the pairs of medical devices that are determined to be connected to each other, a representation of at least one IV line including the pairs of medical devices that are determined to be connected to each other.
Clause 2. The system of clause 1, wherein the at least one processor is programmed and/or configured to determine the pairs of medical devices that are connected to each other by: for each pair of medical devices of the plurality of medical devices, determining, based on the position information associated with that pair of medical devices, a probability that that pair of medical devices are connected to each other.
Clause 3. The system of any of clauses 1 and 2, wherein the at least one processor is programmed and/or configured to determine the pairs of medical devices that are connected to each other by: for each pair of medical devices of the plurality of medical devices, determining, based on the position information associated with that pair of medical devices, the following parameters: a distance between center points associated with that pair of medical devices, an angular difference between orientations of that pair medical devices, and an angle from collinearity of that pair of medical devices, wherein the probability that that pair of medical devices is connected is determined based on the distance between the center points of that pair of medical devices, the angular difference between the orientations of that pair of medical devices, and the angle from collinearity of that pair of medical devices.
Clause 4. The system of any of clauses 1-3, wherein, for each medical device of the plurality of medical devices, that medical device is determined to be connected to another medical device in a pair of medical devices including that medical device that is associated with a highest probability of the pairs of medical devices including that medical device.
Clause 5. The system of any of clauses 1-4, wherein the at least one processor is programmed and/or configured to determine the position information associated with the 3D position of the plurality of medical devices relative to the image capture device by: determining, based on the image, a type of each medical device of the plurality of medical devices, wherein, for a pair of medical devices of the plurality of medical devices, the probability that that pair of medical devices are connected to each other is further determined based on the type of each medical device in that pair of medical devices.
Clause 6. The system of any of clauses 1-5, wherein the at least one processor is programmed and/or configured to determine the pairs of medical devices that are connected to each other by: identifying, based on the type of each medical device of the plurality of medical devices, a set of medical devices of the plurality of medical devices that are associated with a preferred IV line architecture; and for a pair of medical devices included in the set of medical devices associated with the preferred IV line architecture, adjusting a weight used to determine whether the pair of medical devices are connected to each other.
Clause 7. The system of any of clauses 1-6, wherein the at least one processor is programmed and/or configured to determine the pairs of medical devices that are connected to each other by: determining, based on the type of each medical device of the plurality of medical devices, that no medical devices of the plurality of medical devices are associated with a preferred IV line architecture; and in response to determining that no medical devices of the plurality of medical devices are associated with the preferred IV line architecture, prompting a user to obtain another image.
Clause 8. The system of any of clauses 1-7, wherein a first group of medical devices of the plurality of medical devices is associated with a plurality of fiducial markers, wherein the plurality of fiducial markers encapsulates a plurality of identifiers associated with the first group of medical devices and pose information associated with the 3D position of the plurality of fiducial markers, and wherein the at least one processor is programmed and/or configured to determine the position information associated with the 3D position of the plurality of medical devices relative to the image capture device by: determining, based on the image, the plurality of identifiers associated with the first group of medical devices and the pose information associated with the 3D position of the plurality of fiducial markers, wherein, for each medical device of the first group of medical devices, the position information associated with the 3D position of that medical device relative to the image capture device is determined as the 3D position of a fiducial marker associated with that medical device relative to the image capture device.
Clause 9. The system of any of clauses 1-8, wherein the plurality of fiducial markers includes a plurality of AprilTags.
Clause 10. The system of any of clauses 1-9, wherein, for each medical device of the first group of medical devices, the 3D position of the fiducial marker associated with that medical device relative to the image capture device includes x, y, and z coordinates of the fiducial marker and directional vectors for Z, Y, and X axes of the fiducial marker.
Clause 11. The system of any of clauses 1-10, wherein the plurality of identifiers is associated with a plurality of types of medical devices.
Clause 12. The system of any of clauses 1-11, wherein the plurality of identifiers includes a plurality of unique identifiers.
Clause 13. The system of any of clauses 1-12, wherein a second group of medical devices of the plurality of medical devices is not associated with a fiducial marker, and wherein the at least one processor is programmed and/or configured to determine the position information associated with the 3D position of the plurality of medical devices relative to the image capture device by: for each medical device of the second group of medical devices, determining, based on the image, using an object recognition technique, a type of that medical device and the 3D position of that medical device relative to the image capture device.
Clause 14. The system of any of clauses 1-13, wherein the at least one processor is programmed and/or configured to obtain the image of the plurality of medical devices further by: capturing, with the image capture device, the image of the plurality of medical devices.
Clause 15. The system of any of clauses 1-14, wherein the image includes a series of images.
Clause 16. The system of any of clauses 1-15, wherein the image capture device captures the series of images using a burst capture technique.
Clause 17. The system of any of clauses 1-16, wherein the image capture device includes a stereo camera, and wherein the position information associated with the 3D position of the plurality of medical devices relative to the image capture device is determined using a Structure from Motion (SfM) algorithm.
Clause 18. The system of any of clauses 1-17, wherein the image capture device includes a LiDAR system, and wherein the image includes a LiDAR point cloud.
Clause 19. The system of any of clauses 1-18, wherein the at least one processor is programmed and/or configured to generate the representation of the at least one IV line by automatically displaying, on the image, lines connecting the pairs of medical devices that are determined to be connected to each other in the at least on IV line.
Clause 20. The system of any of clauses 1-19, wherein the at least one IV line is associated with at least one pump of an infusion pump in the representation.
Clause 21. The system of any of clauses 1-20, wherein the plurality of fiducial markers is rigidly affixed to rigid portions of the first group of medical devices such that the plurality of fiducial markers cannot translate along and rotate around the first group of medical devices.
Clause 22. The system of any of clauses 1-21, wherein the at least one processor is programmed and/or configured to: determine, based on the pairs of medical devices that are determined to be connected to each other, a dwell time indicating a duration of time a medical device of the plurality of medical devices is connected to another medical device of the plurality of medical devices; and in response to the dwell time satisfying a threshold dwell time, provide, via the user device, an alert associated with the medical device.
Clause 23. A method, comprising: obtaining, with at least one processor, an image of a plurality of medical devices, captured by an image capture device; determining, with at least one processor, based on the image, position information associated with a three-dimensional (3D) position of the plurality of medical devices relative to the image capture device; determining, with the at least one processor, based on the position information, pairs of medical devices of the plurality of medical devices that are connected to each other; and generating, with the at least one processor, based on the pairs of medical devices that are determined to be connected to each other, a representation of at least one IV line including the pairs of medical devices that are determined to be connected to each other.
Clause 24. The method of clause 23, wherein determining the pairs of medical devices that are connected to each other further includes: for each pair of medical devices of the plurality of medical devices, determining, based on the position information associated with that pair of medical devices, a probability that that pair of medical devices are connected to each other.
Clause 25. The method of any of clauses 23 and 24, wherein determining the pairs of medical devices that are connected to each other further includes: for each pair of medical devices of the plurality of medical devices, determining, based on the position information associated with that pair of medical devices, the following parameters: a distance between center points associated with that pair of medical devices, an angular difference between orientations of that pair medical devices, and an angle from collinearity of that pair of medical devices, wherein the probability that that pair of medical devices is connected is determined based on the distance between the center points of that pair of medical devices, the angular difference between the orientations of that pair of medical devices, and the angle from collinearity of that pair of medical devices.
Clause 26. The method of any of clauses 23-25, wherein, for each medical device of the plurality of medical devices, that medical device is determined to be connected to another medical device in a pair of medical devices including that medical device that is associated with a highest probability of the pairs of medical devices including that medical device.
Clause 27. The method of any of clauses 23-26, wherein determining the position information associated with the 3D position of the plurality of medical devices relative to the image capture device further includes: determining, based on the image, a type of each medical device of the plurality of medical devices, wherein, for a pair of medical devices of the plurality of medical devices, the probability that that pair of medical devices are connected to each other is further determined based on the type of each medical device in that pair of medical devices.
Clause 28. The method of any of clauses 23-27, wherein determining the pairs of medical devices that are connected to each other further includes: identifying, based on the type of each medical device of the plurality of medical devices, a set of medical devices of the plurality of medical devices that are associated with a preferred IV line architecture; and for a pair of medical devices included in the set of medical devices associated with the preferred IV line architecture, adjusting a weight used to determine whether the pair of medical devices are connected to each other.
Clause 29. The method of any of clauses 23-28, wherein determining the pairs of medical devices that are connected to each other further includes: determining, based on the type of each medical device of the plurality of medical devices, that no medical devices of the plurality of medical devices are associated with a preferred IV line architecture; and in response to determining that no medical devices of the plurality of medical devices are associated with the preferred IV line architecture, prompting a user to obtain another image.
Clause 30. The method of any of clauses 23-29, wherein a first group of medical devices of the plurality of medical devices is associated with a plurality of fiducial markers, wherein the plurality of fiducial markers encapsulates a plurality of identifiers associated with the first group of medical devices and pose information associated with the 3D position of the plurality of fiducial markers, and wherein determining the position information associated with the 3D position of the plurality of medical devices relative to the image capture device includes: determining, based on the image, the plurality of identifiers associated with the first group of medical devices and the pose information associated with the 3D position of the plurality of fiducial markers, wherein, for each medical device of the first group of medical devices, the position information associated with the 3D position of that medical device relative to the image capture device is determined as the 3D position of a fiducial marker associated with that medical device relative to the image capture device.
Clause 31. The method of any of clauses 23-30, wherein the plurality of fiducial markers includes a plurality of AprilTags.
Clause 32. The method of any of clauses 23-31, wherein, for each medical device of the first group of medical devices, the 3D position of the fiducial marker associated with that medical device relative to the image capture device includes x, y, and z coordinates of the fiducial marker and directional vectors for Z, Y, and X axes of the fiducial marker.
Clause 33. The method of any of clauses 23-32, wherein the plurality of identifiers is associated with a plurality of types of medical devices.
Clause 34. The method of any of clauses 23-33, wherein the plurality of identifiers includes a plurality of unique identifiers.
Clause 35. The method of any of clauses 23-34, wherein a second group of medical devices of the plurality of medical devices is not associated with a fiducial marker, and wherein determining the position information associated with the 3D position of the plurality of medical devices relative to the image capture device further includes: for each medical device of the second group of medical devices, determining, based on the image, using an object recognition technique, a type of that medical device and the 3D position of that medical device relative to the image capture device.
Clause 36. The method of any of clauses 23-35, wherein obtaining the image of the plurality of medical devices further includes: capturing, with the image capture device, the image of the plurality of medical devices.
Clause 37. The method of any of clauses 23-36, wherein the image includes a series of images.
Clause 38. The method of any of clauses 23-37, wherein the image capture device captures the series of images using a burst capture technique.
Clause 39. The method of any of clauses 23-38, wherein the image capture device includes a stereo camera, and wherein the position information associated with the 3D position of the plurality of medical devices relative to the image capture device is determined using a Structure from Motion (SfM) algorithm.
Clause 40. The method of any of clauses 23-39, wherein the image capture device includes a LiDAR system, and wherein the image includes a LiDAR point cloud.
Clause 41. The method of any of clauses 23-40, wherein generating the representation of the at least one IV line includes automatically displaying, on the image, lines connecting the pairs of medical devices that are determined to be connected to each other in the at least on IV line.
Clause 42. The method of any of clauses 23-41, wherein the at least one IV line is associated with at least one pump of an infusion pump in the representation.
Clause 43. The method of any of clauses 23-42, wherein the plurality of fiducial markers is rigidly affixed to rigid portions of the first group of medical devices such that the plurality of fiducial markers cannot translate along and rotate around the first group of medical devices.
Clause 44. The method of any of clauses 23-43, further comprising: determining, with the at least one processor, based on the pairs of medical devices that are determined to be connected to each other, a dwell time indicating a duration of time a medical device of the plurality of medical devices is connected to another medical device of the plurality of medical devices; and in response to the dwell time satisfying a threshold dwell time, providing, with the at least one processor, via the user device, an alert associated with the medical device.

### BRIEF DESCRIPTION OF THE DRAWINGS

Additional advantages and details are explained in greater detail below with reference to the exemplary embodiments that are illustrated in the accompanying schematic figures, in which:
FIG. 1A is a diagram of non-limiting embodiments or aspects of an environment in which systems, devices, products, apparatus, and/or methods, described herein, can be implemented;
FIG. 1B is a diagram of non-limiting embodiments or aspects of an implementation of an environment in which systems, devices, products, apparatus, and/or methods, described herein, can be implemented;
FIG. 2 is a diagram of non-limiting embodiments or aspects of components of one or more devices and/or one or more systems of FIGS. 1A and 1B;
FIG. 3A is a perspective view of implementations of non-limiting embodiments or aspects of medical devices;
FIG. 3B is a perspective view of the implementations of non-limiting embodiments or aspects of medical devices in FIG. 3A connected together;
FIG. 4 is a flow chart of non-limiting embodiments or aspects of a process for vascular access management;
FIG. 5 shows an example image of a catheter insertion site including a plurality of medical devices.
FIG. 6 illustrates an implementation of non-limiting embodiments or aspects of a fiducial marker;
FIG. 7 is a perspective view of an example image of a catheter insertion site on a patient;
FIG. 8 illustrates example parameters used in an implementation of non-limiting embodiments or aspects of a process for vascular access management;
FIG. 9 is a chart of example unlikely but possible connections between medical devices;
FIG. 10 illustrates an implementation of non-limiting embodiments or aspects of a representation of IV lines; and
FIG. 11 is illustrates an example catheter tree building sequence of a process for vascular access management according to non-limiting embodiments or aspects.

### DETAILED DESCRIPTION

It is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects. Hence, specific dimensions and other physical characteristics related to the embodiments or aspects disclosed herein are not to be considered as limiting.

For purposes of the description hereinafter, the terms "end," "upper," "lower," "right," "left," "vertical," "horizontal," "top," "bottom," "lateral," "longitudinal," and derivatives thereof shall relate to embodiments or aspects as they are oriented in the drawing figures. However, it is to be understood that embodiments or aspects may assume various alternative variations and step sequences, except where expressly specified to the contrary. It is also to be understood that the specific devices and processes illustrated in the attached drawings, and described in the following specification, are simply non-limiting exemplary embodiments or aspects. Hence, specific dimensions and other physical characteristics related to the embodiments or aspects of the embodiments or aspects disclosed herein are not to be considered as limiting unless otherwise indicated.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise.

As used herein, the terms "communication" and "communicate" may refer to the reception, receipt, transmission, transfer, provision, and/or the like of information (e.g., data, signals, messages, instructions, commands, and/or the like). For one unit (e.g., a device, a system, a component of a device or system, combinations thereof, and/or the like) to be in communication with another unit means that the one unit is able to directly or indirectly receive information from and/or transmit information to the other unit. This may refer to a direct or indirect connection that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit (e.g., a third unit located between the first unit and the second unit) processes information received from the first unit and communicates the processed information to the second unit. In some non-limiting embodiments or aspects, a message may refer to a network packet (e.g., a data packet and/or the like) that includes data. It will be appreciated that numerous other arrangements are possible.

As used herein, the term "computing device" may refer to one or more electronic devices that are configured to directly or indirectly communicate with or over one or more networks. A computing device may be a mobile or portable computing device, a desktop computer, a server, and/or the like. Furthermore, the term "computer" may refer to any computing device that includes the necessary components to receive, process, and output data, and normally includes a display, a processor, a memory, an input device, and a network interface. A "computing system" may include one or more computing devices or computers. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.). Further, multiple computers, e.g., servers, or other computerized devices directly or indirectly communicating in the network environment may constitute a "system" or a "computing system".

It will be apparent that systems and/or methods, described herein, can be implemented in different forms of hardware, software, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

Some non-limiting embodiments or aspects are described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, etc.

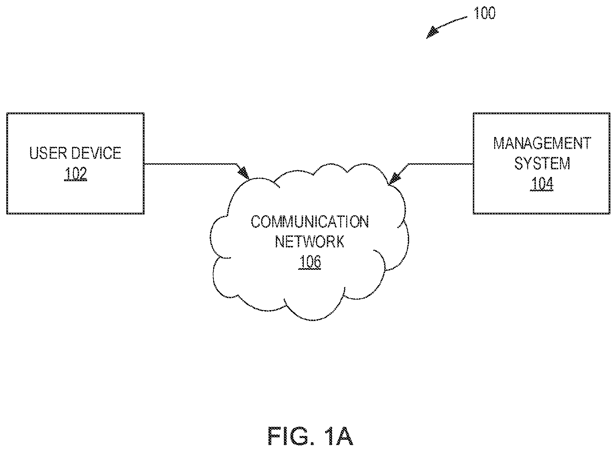

Referring now to FIG. 1A, FIG. 1A is a diagram of an example environment 100 in which devices, systems, methods, and/or products described herein, may be implemented. As shown in FIG. 1A, environment 100 includes user device 102, management system 104, and/or communication network 106. Systems and/or devices of environment 100 can interconnect via wired connections, wireless connections, or a combination of wired and wireless connections.

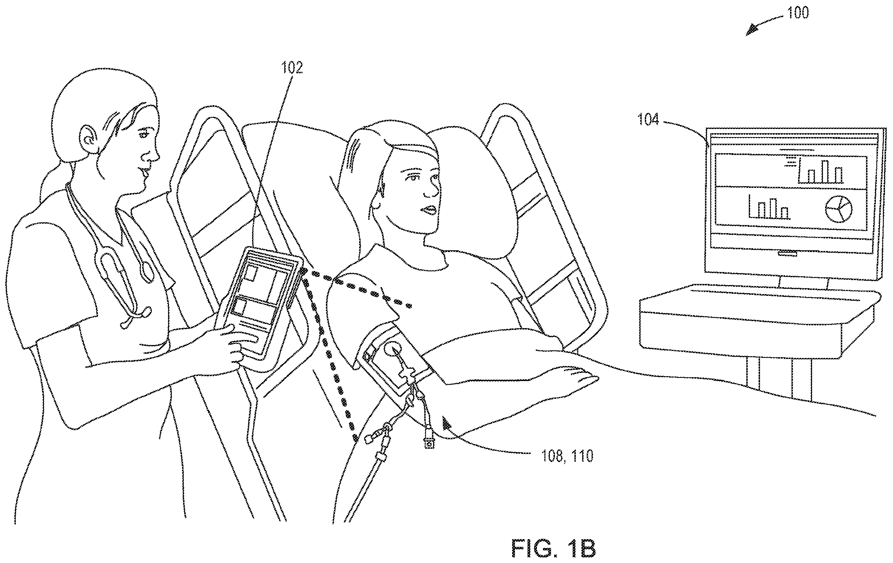

Referring also to FIG. 1B, FIG. 1B is a diagram of non-limiting embodiments or aspects of an implementation of environment 100 in which systems, devices, products, apparatus, and/or methods, described herein, can be implemented. For example, as shown in FIG. 1B, environment 100 may include a hospital room including a patient, one or more medical devices 108, one or more fiducial markers 110 associated with the one or more medical devices 108, and/or a caretaker (e.g., a nurse, etc.).

User device 102 may include one or more devices capable of receiving information and/or data from management system 104 (e.g., via communication network 106, etc.) and/or communicating information and/or data to management system 104 (e.g., via communication network 106, etc.). For example, user device 102 may include one or more computing systems including one or more processors (e.g., one or more computing devices, one or more server computers, one or more mobile computing devices, one or more tablet computers, etc.). In some non-limiting embodiments or aspects, user device 102 may include a tablet computer or mobile computing device, such as an Apple^{®} iPad, an Apple^{®} iPhone, an Android^{®} tablet, an Android^{®} phone, and/or the like.

User device 102 may include one or more image capture devices (e.g., one or more cameras, one or more sensors, etc.) configured to capture one or more images of an environment (e.g., environment 100, etc.) surrounding the one or more image capture devices. For example, user device 102 may include one or more image capture devices configured to capture one or more images of the one or more medical devices 108, the one or more fiducial markers 110 associated with the one or more medical devices 108, and/or the patient. As an example, user device 102 may include at least one of the following image capture devices: a camera, a stereo camera, a LiDAR sensor, or any combination thereof.

Management system 104 may include one or more devices capable of receiving information and/or data from user device 102 (e.g., via communication network 106, etc.) and/or communicating information and/or data to user device 102 (e.g., via communication network 106, etc.). For example, management system 104 may include one or more computing systems including one or more processors (e.g., one or more computing devices, one or more server computers, one or more mobile computing devices, etc.). In some non-limiting embodiments or aspects, management system 104 includes and/or is accessible via a nurse station or terminal in a hospital. For example, management system 104 may provide bedside nurse support, nursing station manager support, retrospective reporting for nursing administration, and/or the like.

Communication network 106 may include one or more wired and/or wireless networks. For example, communication network 106 may include a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN)), a private network, an ad hoc network, an intranet, the Internet, a fiber optic-based network, a cloud computing network, and/or the like, and/or a combination of these or other types of networks.

The number and arrangement of systems and devices shown in FIGS. 1A and 1B are provided as an example. There can be additional systems and/or devices, fewer systems and/or devices, different systems and/or devices, or differently arranged systems and/or devices than those shown in FIGS. 1A and 1B. Furthermore, two or more systems or devices shown in FIGS. 1A and 1B can be implemented within a single system or a single device, or a single system or a single device shown in FIGS. 1A and 1B can be implemented as multiple, distributed systems or devices. Additionally, or alternatively, a set of systems or a set of devices (e.g., one or more systems, one or more devices, etc.) of environment 100 can perform one or more functions described as being performed by another set of systems or another set of devices of environment 100.

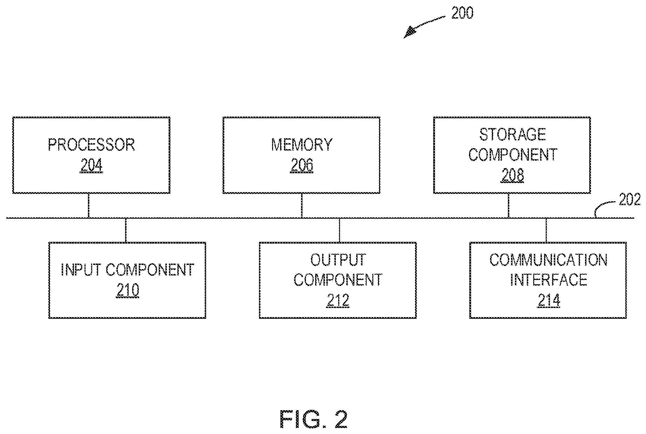

Referring now to FIG. 2, FIG. 2 is a diagram of example components of a device 200. Device 200 may correspond to user device 102 (e.g., one or more devices of a system of user device 102, etc.) and/or one or more devices of management system 104. In some non-limiting embodiments or aspects, user device 102 (e.g., one or more devices of a system of user device 102, etc.) and/or one or more devices of management system 104 may include at least one device 200 and/or at least one component of device 200. As shown in FIG. 2, device 200 may include bus 202, processor 204, memory 206, storage component 208, input component 210, output component 212, and communication interface 214.

Bus 202 may include a component that permits communication among the components of device 200. In some non-limiting embodiments or aspects, processor 204 may be implemented in hardware, software, or a combination of hardware and software. For example, processor 204 may include a processor (e.g., a central processing unit (CPU), a graphics processing unit (GPU), an accelerated processing unit (APU), etc.), a microprocessor, a digital signal processor (DSP), and/or any processing component (e.g., a field-programmable gate array (FPGA), an application-specific integrated circuit (ASIC), etc.) that can be programmed to perform a function. Memory 206 may include random access memory (RAM), read-only memory (ROM), and/or another type of dynamic or static storage device (e.g., flash memory, magnetic memory, optical memory, etc.) that stores information and/or instructions for use by processor 204.

Storage component 208 may store information and/or software related to the operation and use of device 200. For example, storage component 208 may include a hard disk (e.g., a magnetic disk, an optical disk, a magneto-optic disk, a solid state disk, etc.), a compact disc (CD), a digital versatile disc (DVD), a floppy disk, a cartridge, a magnetic tape, and/or another type of computer-readable medium, along with a corresponding drive.

Input component 210 may include a component that permits device 200 to receive information, such as via user input (e.g., a touch screen display, a keyboard, a keypad, a mouse, a button, a switch, a microphone, etc.). Additionally or alternatively, input component 210 may include a sensor for sensing information (e.g., a global positioning system (GPS) component, an accelerometer, a gyroscope, an actuator, etc.). Output component 212 may include a component that provides output information from device 200 (e.g., a display, a speaker, one or more light-emitting diodes (LEDs), etc.).

Communication interface 214 may include a transceiver-like component (e.g., a transceiver, a separate receiver and transmitter, etc.) that enables device 200 to communicate with other devices, such as via a wired connection, a wireless connection, or a combination of wired and wireless connections. Communication interface 214 may permit device 200 to receive information from another device and/or provide information to another device. For example, communication interface 214 may include an Ethernet interface, an optical interface, a coaxial interface, an infrared interface, a radio frequency (RF) interface, a universal serial bus (USB) interface, a Wi-Fi^{®} interface, a cellular network interface, and/or the like.

Device 200 may perform one or more processes described herein. Device 200 may perform these processes based on processor 204 executing software instructions stored by a computer-readable medium, such as memory 206 and/or storage component 208. A computer-readable medium (e.g., a non-transitory computer-readable medium) is defined herein as a non-transitory memory device. A memory device includes memory space located inside of a single physical storage device or memory space spread across multiple physical storage devices.

Software instructions may be read into memory 206 and/or storage component 208 from another computer-readable medium or from another device via communication interface 214. When executed, software instructions stored in memory 206 and/or storage component 208 may cause processor 204 to perform one or more processes described herein. Additionally or alternatively, hardwired circuitry may be used in place of or in combination with software instructions to perform one or more processes described herein. Thus, embodiments or aspects described herein are not limited to any specific combination of hardware circuitry and software.

Memory 206 and/or storage component 208 may include data storage or one or more data structures (e.g., a database, etc.). Device 200 may be capable of receiving information from, storing information in, communicating information to, or searching information stored in the data storage or one or more data structures in memory 206 and/or storage component 208.

The number and arrangement of components shown in FIG. 2 are provided as an example. In some non-limiting embodiments or aspects, device 200 may include additional components, fewer components, different components, or differently arranged components than those shown in FIG. 2. Additionally or alternatively, a set of components (e.g., one or more components) of device 200 may perform one or more functions described as being performed by another set of components of device 200.

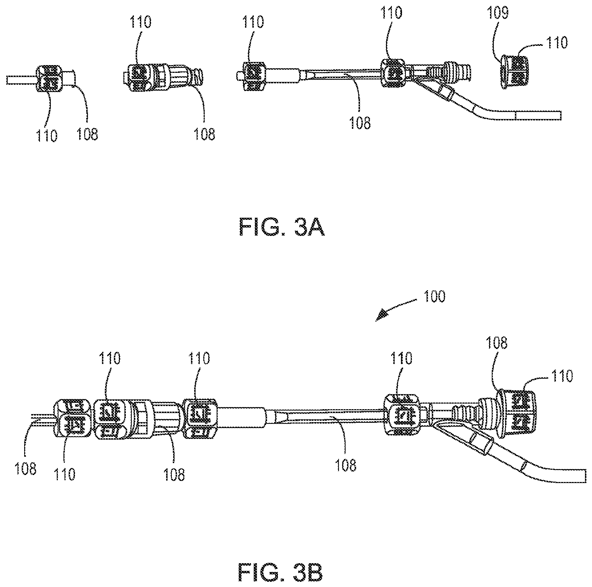

Referring now to FIGS. 3A and 3B, FIG. 3A is a perspective view of implementations of non-limiting embodiments or aspects of medical devices, and FIG. 3B is a perspective view of the implementations of non-limiting embodiments or aspects of medical devices in FIG. 3A connected together.

A medical device 108 may include at least one of the following types of medical devices: a peripheral IV catheter (PIVC), a peripherally inserted central catheter (PICC), a midline catheter, a central venous catheter (CVC), a needleless connector, a catheter dressing, a catheter stabilization device, a disinfectant cap, a disinfectant swab or wipe, an IV tubing set, an extension set, a Y connector, a stopcock, an infusion pump, a flush syringe, a medication delivery syringe, an IV fluid bag, a lumen adapter (e.g., a number of lumen adapters associated with a catheter may indicate a number of lumens included in the catheter, etc.), or any combination thereof.

A fiducial marker 110 (e.g., a tag, a label, a code, etc.) may be associated with (e.g., removably attached to, permanently attached to, integrated with, implemented on, etc.) a medical device 108. In some non-limiting embodiments or aspects, each medical device 108 in environment 100 may be associated with a fiducial marker 110. In some non-limiting embodiments or aspects, only a portion the medical devices 108 in environment 100 may be associated with fiducial markers 110. In some non-limiting embodiments or aspects, none of the medical devices 108 in environment 100 may be associated with a fiducial marker 110.

A fiducial marker 110 may encapsulate an identifier associated with a type of a medical device 108 associated with the fiducial marker 110 and/or uniquely identify the medical device 108 associated with the fiducial marker 110 from other medical devices. For example, a fiducial marker 110 may encapsulate an identifier associated with at least one of the following types of medical devices: a peripheral IV catheter (PIVC), a peripherally inserted central catheter (PICC), a midline catheter, a central venous catheter (CVC), a needleless connector, a disinfectant cap, a disinfectant swab or wipe, an IV tubing set, an extension set, a Y connector, a stopcock, an infusion pump, a flush syringe, a medication delivery syringe, an IV fluid bag, or any combination thereof, and/or uniquely identify a medical device 108 (e.g., a first needless connector, etc.) from other medical devices (e.g., a second needless connector, etc.) including identifiers associated with a same type of medical device.

A fiducial marker 110 may encapsulate pose information associated with a 3D position of the fiducial marker 110. For example, fiducial marker 110 may include markings that, when captured in an image, enable computing a precise 3D position of the fiducial marker with respect to the image capture device that captured the image (e.g., an x, y, z coordinate position of the fiducial marker, etc.) and/or a precise 2D position of the fiducial marker in the image itself (e.g., a x, y coordinate positon of the fiducial marker in the image, etc.).

In some non-limiting embodiments or aspects, a fiducial marker 110 may include an AprilTag. For example, a fiducial marker 110 may include an AprilTag V3 of type customTag 48h12, which enables using AprilTag V3 detection to determine a unique ID, which may indicate a type of the medical device 108 associated with the fiducial marker (e.g., in leading digits, etc.) and/or a unique serial number for that specific medical device 108 (e.g., in the trailing digits, etc.), and/or a location (e.g., x, y, and z coordinates, directional vectors for Z, Y, and X axes, etc.) of the fiducial marker 110 in a field-of-view (FOV) of an image capture device. However, non-limiting embodiments or aspects are not limited thereto, and a fiducial marker 110 may include a QR code, a barcode (e.g., a 1D barcode, a 2D barcode, etc.), an Aztec code, a Data Matrix code, an ArUco marker, a colored pattern, a reflective pattern, a fluorescent pattern, a predetermined shape and/or color (e.g., a red pentagon, a blue hexagon, etc.), an LED pattern, a hologram, and/or the like that encapsulates an identifier associated with a type of a medical device 108 associated with the fiducial marker 110, uniquely identifies the medical device 108 associated with the fiducial marker 110 from other medical devices, and/or encapsulates pose information associated with a 3D position of the fiducial marker 110.

In some non-limiting embodiments or aspects, a fiducial marker 110 may include color calibration areas or regions positioned adjacent to variable color regions to calibrate color in a wider range of lighting conditions. For example, for a 2x2 grid, a cell (1,1) in an upper-left corner of the gird may include a predetermined and/or standard calibration color region (e.g., neutral gray, etc.), and user device 102 and/or management system 104 may use the predetermined and/or standard calibration color region to calibrate colors in images used to detect or determine the fiducial marker 110 in those images and/or to detect or determine color changes in tissue of a patient (e.g., patient tissue adjacent an insertion site, etc.) in those images. In such an example, user device 102 and/or management system 104 may use the predetermined and/or standard calibration color region to orient the fiducial marker 110 to determine how to properly rotate and decode the colors in the fiducial marker 110 to decode the identifier encapsulated by the fiducial marker 110 and/or track the fiducial marker 110 within environment 100.

As shown in FIGS. 3A and 3B, fiducial markers 110 may be arranged symmetrically in rings about an axis of a medical device 108 associated with those fiducial markers, which may enable at least one fiducial marker 110 being presented to the FOV of an image capture device regardless of an orientation of the medical device 108. A fiducial marker 110 may be clocked such that a direction of the marker (e.g., as indicated by the pose information thereof, etc.) aligns with the proximal or distal direction of fluid flow through a medical device 108 associated with that fiducial marker. A fiducial marker 110 may be rigidly affixed to a medical device 108 such that the fiducial marker 110 cannot translate along and/or rotate around the medical device 108 (e.g., rigidly affixed to a rigid portion of a medical device 108 and/or a catheter tree including the medical device 108, etc.), which may reduce movement and/or changes in distance of the fiducial marker relative to other fiducial markers and/or medical devices.

Fiducial markers 110 may be located at or directly adjacent to connection points or ports of each medical device 108, such that the fiducial markers 110 on connected medical devices 108 are collinear (e.g., parallel, etc.). For example, fiducial markers 110 on connected medical devices 108 that are collinear (e.g., parallel, etc.) in this way may also be contiguous, or at a known distance apart.

A single medical device 108 may include one or more sets of fiducial markers 110. For example, as shown in FIGS. 3A and 3B, the cap at the far right of each of these figures includes a single set of fiducial markers 110 (e.g., with each fiducial marker 110 in the set being identical and/or encapsulating the same information, etc.), and the tubing directly to the left of the cap includes two sets of fiducial markers 110 at respective connection points of the tubing and separated by the tubing. In such an example, collinearity between the fiducial markers 110 at each end of the tubing may not be guaranteed, and connection between the fiducial markers 110 at each end of the tubing may be established via a pre-defined scheme (e.g., with each fiducial marker 110 in each set of fiducial markers 110 on the same medical device 110 having a same value or different but contiguous values, etc.). It is noted that spacing between connected medical devices 108 may vary (e.g., as shown on the left in FIG. 3B, etc.); however, this spacing may be deterministic and known by user device 102 and/or management system 104 for each possible connection between medical devices.

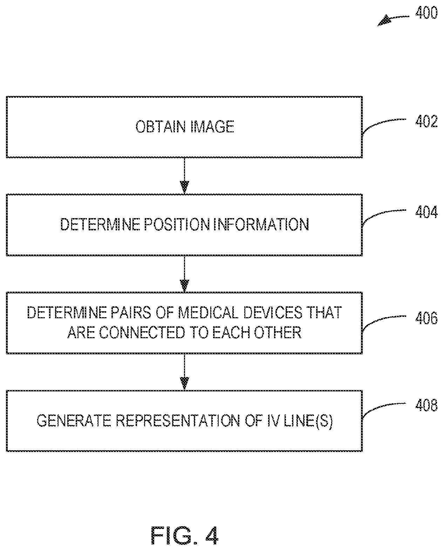

Referring now to FIG. 4, FIG. 4 is a flowchart of non-limiting embodiments or aspects of a process 400 for vascular access management. In some non-limiting embodiments or aspects, one or more of the steps of process 400 may be performed (e.g., completely, partially, etc.) by user device 102 (e.g., one or more devices of a system of user device 102, etc.). In some non-limiting embodiments or aspects, one or more of the steps of process 400 may be performed (e.g., completely, partially, etc.) by another device or a group of devices separate from or including user device 102, such as management system 104 (e.g., one or more devices of management system 104, etc.).

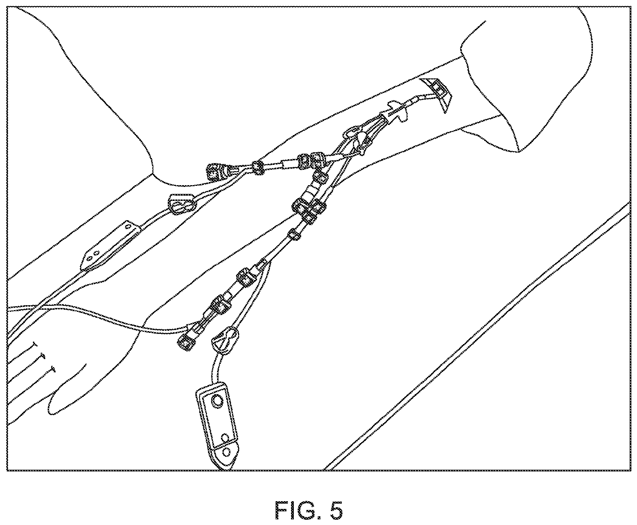

As shown in FIG. 4, at step 402, process 400 includes obtaining an image. For example, user device 102 may obtain an image (e.g., a single image, a plurality of images, a series of images, etc.) of a plurality of medical devices 108, captured by an image capture device. As an example, an image capture device of user device 102 may capture the image of the plurality of medical devices 108. In such an example, a nurse may use user device 102 to take one or more images of a catheter site of a patient and/or an infusion pump connected to the catheter site. For example, FIG. 5 shows an example image of a catheter insertion site including a plurality of medical devices.

In some non-limiting embodiments or aspects, an image may include a series of images. For example, an image capture device of user device 102 may capture the series of images using a burst capture technique (e.g., a "burst mode", a continuous shooting mode, etc.), which may enable user device 102 to create a second likelihood layer for determining probabilities that pairs of medical devices 108 are connected as described herein below in more detail, thereby refining motion artifacts, angles, distances, and/or missed fiducial marker detection. As an example, an image capture device of user device 102 may capture the series of images as a live video feed to identify pairs of medical devices 108 that are connected to each other (e.g., to identify catheter tree components and generate a catheter tree, etc.) as the live video feed is captured.

As shown in FIG. 4, at step 404, process 400 includes determining position information. For example, user device 102 may determine, based on the image, position information associated with a 3D position of the plurality of medical devices 108 relative to the image capture device and/or a 2D position of the medial device 108 in the image itself. In such an example, determining the position information associated with the 3D position of the plurality of medical devices 108 relative to the image capture device and/or the 2D position of the plurality of medical devices 108 in the image itself may further include determining, based on the image, a type of each medical device of the plurality of medical devices 108.

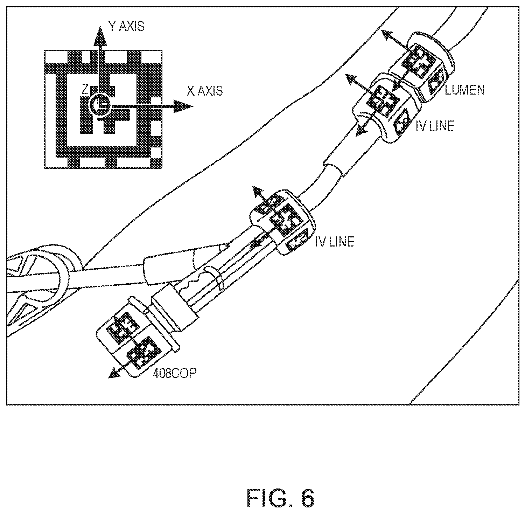

In some non-limiting embodiments or aspects, a first group of medical devices of the plurality of medical devices 108 is associated with a plurality of fiducial markers 110. The plurality of fiducial markers 110 may encapsulate a plurality of identifiers associated with the first group of medical devices and pose information associated with the 3D position and/or 2D position of the plurality of fiducial markers 110. In such an example, user device 102 may determine the position information associated with the 3D position of the first group of medical devices relative to the image capture device and/or the 2D position of the plurality of medical devices 108 in the image itself by determining or identifying, based on the image, the plurality of identifiers associated with the first group of medical devices and the pose information associated with the 3D positions and/or 2D positions of the plurality of fiducial markers 110. For example, and referring also to FIG. 6, the plurality of fiducial markers 110 may include a plurality of AprilTags, and user device 102 may process the image using AprilTag detection software to determine types of the medical devices 108 associated with the fiducial markers 110 and/or a unique serial number for the specific medical devices 108 and to compute a precise 3D position, orientation, and/or identity of the plurality of fiducial markers 110 relative to the image capture device that captured the image and/or a precise 2D position of the plurality of fiducial markers 110 in the image itself. As an example, for each medical device 108 of the first group of medical devices, the position information associated with the 3D position of that medical device 108 relative to the image capture device may be determined as the 3D position of a fiducial marker 110 associated with that medical device 108 relative to the image capture device and/or the position information associated with the 2D positon of that medical device 108 in the image itself may be determined as the 2D position of the fiducial marker 110 associated with that medical device 110. In such an example, for each medical device 108 of the first group of medical devices, the 3D position of the fiducial marker 110 associated with that medical device relative to the image capture device may include x, y, and z coordinates of the fiducial marker 110 and/or directional vectors for Z, Y, and X axes of the fiducial marker 110. In such an example, for each medical device 108 of the first group of medical devices, the 2D position of the fiducial marker 110 in the image itself may include x, y coordinates of the fiducial marker 110 in the image and/or direction vectors for Y and X axe of the fiducial marker.

In some non-limiting embodiments or aspects, a second group of medical devices of the plurality of medical devices is not associated with a fiducial marker. In such an example, user device 102 may determine the position information associated with the 3D position of the second group of medical devices relative to the image capture device and/or the 2D position of the second group of medical devices in the image itself by determining or identifying, for each medical device of the second group of medical devices, based on the image, using one or more existing object detection techniques, a type of that medical device, the 3D position of that medical device relative to the image capture device, and/or the 2D position of that medical device in the image itself. For example, medical devices 108 without fiducial markers or identifier tags can be identified by user device 102 processing the image using one or more object detection techniques (e.g., a deep learning technique, an image processing technique, an image segmentation technique, etc.) to identify or determine medical devices 108 in the image and the position information associated with the 3D position of the identified medical devices 108 relative to the image capture device (e.g., include x, y, and z coordinates of the medical devices and directional vectors for Z, Y, and X axes of the medical devices 108, etc.) and/or the 2D positions of the identified medical devices in the image itself. For example, a deep learning technique may include a bounding box technique that generates a box label for objects (e.g., medical devices 108, etc.) of interest in images, an image masking technique (e.g., masked FRCNN (RCNN or CNN) that captures specific shapes of objects (e.g., medical devices 108, etc.) in images, a trained neural network that identifies objects (e.g., medical devices 108, etc.) in images, a classifier that classifies identified objects into classes or type of the objects, and/or the like. As an example, an image processing technique may include a cross correlation image processing technique, an image contrasting technique, a binary or colored filtering technique, and/or the like. As an example, different catheter lumens may include unique colors that can be used by the image processing to identify a type of the catheter.

In some non-limiting embodiments or aspects, user device 102 may process the image data using a stereoscopic imaging technique and/or a shadow distance technique to determine object data including a distance from the image capture system to detected objects and/or distances between detected objects, and/or image capture system 102 may obtain the image data using multiple cameras, a laser focus technology, LiDAR sensors, and/or a camera physical zoom-in function to determine object data including a distance from the image capture system to detected objects and/or distances between detected objects. In some non-limiting embodiments or aspects, image capture system 102 may obtain image data and/or object data including a 3D profile of an object using a 3D optical profiler.

For example, an image capture device may include a stereo camera, and/or the position information associated with the 3D position of the plurality of medical devices relative to the image capture device may be determined using a Structure from Motion (SfM) algorithm. As an example, user device 102 may include a stereo camera setup, which is available in many mobiles devices, such Apple^{®} iPads, Apple^{®} iPhones, Android^{®} tablets, Android^{®} phones, and/or the like. User device 102 may process images from the stereo camera using SfM algorithms to extract 3D information which may enhance the object feature recognition of the fiducial markers 110 of the first group of medical devices and/or of the medical devices in the second group of medical devices without fiducial markers. As an example, the SfM processing may improve extraction of the 3D features from the medical devices in the second group of medical devices without fiducial markers, which may improve chances of image feature accumulation, e.g., by using a burst image capture/video mode which captures images in a proper direction/registration (e.g., pan/tilt, etc.) based on the setup or locations of the medical devices 108 and/or an anatomical position thereof on the body of the patient, with a catheter tree being built by starting from a dressing tag and connecting center-points or centroids of medical devices 108 detected using object recognition techniques to other medical device 108 with or without fiducial markers 110. As another example, these 3D features extracted using the SfM processing may be utilized for a calculation of a co-planarity of medical devices 108 (e.g., for generating a catheter tree, etc.), which may provide an added advantage on a multi-lumen & multi-tubing catheter setup by reducing missed connections that may occur in a 2D single plane space due to false co-linearity.

For example, an image capture device may include a LiDAR system, and the image may include a LiDAR point cloud. As an example, user device 102 may include a mini-LiDAR system, which is available in many mobiles devices, such Apple^{®} iPads, Apple^{®} iPhones, Android^{®} tablets, Android^{®} phones, and/or the like. In such an example, use of LiDAR images may improve accuracy for 3D feature detection because LiDAR images directly provide 3D world information as point clouds which may accelerate the 3D data collection with reduced or minimum protocols when compared to stereo setup. For example, user device 102 may use existing image registration and/or transformation techniques to overlap 2D object information from camera images, such as color, texture and/or the like, with the 3D LiDAR point cloud to detect 3D features for enhancing catheter tree generation and connection accuracy, which may also improve augmented reality generation and environment restructuring, as well as guide the catheter tree-generation and connectivity determination.

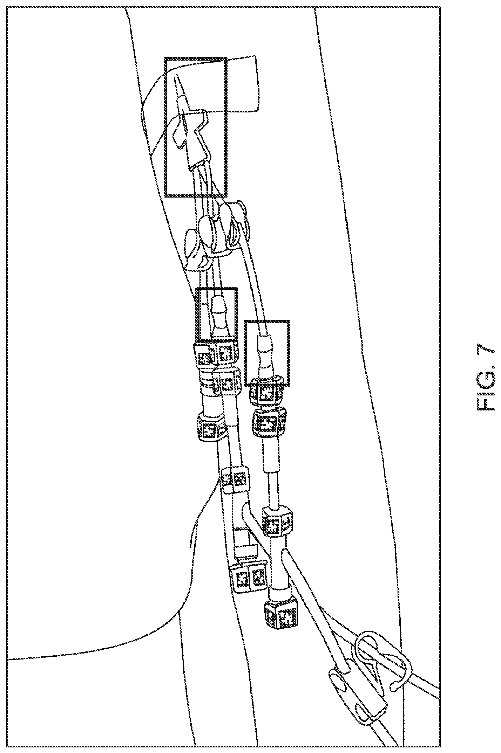

For example, and referring also to FIG. 7, which is a perspective view of an example catheter insertion site on a patient, due to a positioning of the AprilTags and the different tubing, there may be a high chance for tree generation mismatch (e.g., incorrect determination of connections between medical devices, etc.) as the fiducial markers 110 and/or the medical devices 108 appear to be co-linear in 2D due to their proximity. User device 102 may use the above-described stereo image/SfM and/or LiDAR image based approaches for determining 3D features of the fiducial markers 110 and/or the medical devices 108 to improve the co-planar information and/or provide more accurate catheter tree generation (e.g., more accurate determination of connections between devices, etc.).

As shown in FIG. 4, at step 406, process 400 includes determining pairs of medical devices that are connected to each other. For example, user device 102 may determine, based on the position information and/or the types of the plurality of medical devices 108, pairs of medical devices of the plurality of medical devices 108 that are connected to each other. As an example, for each pair of medical devices of the plurality of medical devices 108, user device 102 may determine, based on the 3D position information associated with that pair of medical devices, the 2D position information associated with that pair of medical devices, and/or the types of that pair of medical devices, a probability that that pair of medical devices are connected to each other. In such an example, for each medical device of the plurality of medical devices 108, that medical device may be determined to be connected to another medical device in a pair of medical devices including that medical device that is associated with a highest probability of the pairs of medical devices including that medical device.

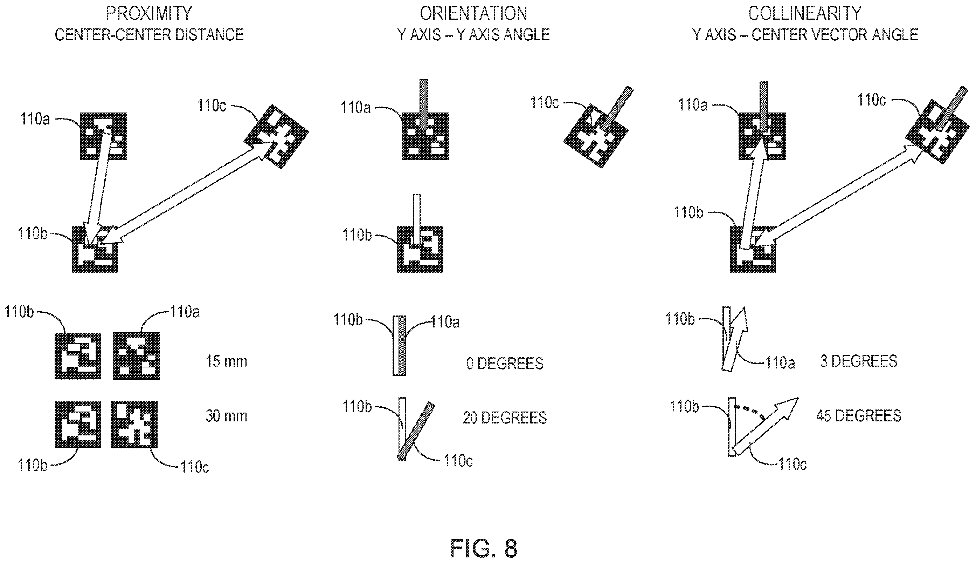

For example, for each pair of medical devices of the plurality of medical devices, user device 102 may determine, based on the position information associated with that pair of medical devices, the following parameters: a distance between center points associated with that pair of medical devices, an angular difference between orientations of that pair medical devices, and/or an angle from collinearity of that pair of medical devices, and the probability that that pair of medical devices is connected may be determined based on the distance between the center points of that pair of medical devices, the angular difference between the orientations of that pair of medical devices, and/or the angle from collinearity of that pair of medical devices. As an example, and referring also to FIG. 8, for each pair of fiducial markers of the plurality of fiducial markers 110a, 110b, and 110c, user device 102 may determine, based on the 3D position information associated with that pair of fiducial markers and/or the 2D position information, the following parameters: a distance between center points of that pair of fiducial markers (e.g., a proximity, etc.), an angular difference between orientations of that pair of fiducial markers (e.g., an orientation, etc.), and/or an angle from collinearity of that pair of fiducial markers (e.g., a collinearity, etc.), and the probability that the pair of medical devices associated with that pair of fiducial markers is connected may be determined based on the determined proximity, orientation, and/or collinearity (e.g., an angular difference in orientation and angle created by tag orientation and the connection vector, etc.). Although shown in FIG. 8 as determined between pairs of fiducial markers 110, non-limiting embodiments or aspects are not limited thereto and the proximity, orientation, and/or collinearity between medical devices may be determined as between pairs of medical devices without fiducial markers and/or between pairs of medical devices including a single medical device associated with a fiducial marker and a single medical device without a fiducial marker.

In some non-limiting embodiments or aspects, user device 102 may use a probability-based tree building logic or a ruleset (e.g., a predetermine ruleset, etc.) to determine the pair of medical devices 108 that are connected to each other. As an example, user device 102 may determine the probabilities for each medical device 108 toward each of the other medical devices using the above-described parameters of proximity, orientation, and/or collinearity (and/or one or more additional parameters, such as X and Y axes distances between the pair of medical devices, a difference in depth from the image capture device between the pair of medical devices, and/or the like), and user device 102 may give each parameter a predefined weight toward a total probability that the pair of medical devices are connected to each other (e.g., a sum of each parameter weight may be 1, etc.) when applying the probability-based tree building logic or ruleset to determine the pairs of medical devices 108 that are connected to each other. In such an example, user device 102 may process the pairs of medical devices 108 by using a dressing tag and/or a lumen adapter as a starting point or anchor for generation of a catheter tree or representation of the IV line(s), and, for each medical device, that medical device may be determined to be connected to the other medical device in the pair of medical devices for which that medical device has a highest connection probability.

In such an example, user device 102 may identify, based on the type of each medical device of the plurality of medical devices, a set of medical devices of the plurality of medical devices that are associated with a preferred IV line architecture. For a pair of medical devices included in the set of medical devices associated with the preferred IV line architecture, user device 102 may adjust a weight used to determine whether the pair of medical devices are connected to each other (e.g., a known preferred architecture may receive a higher weight in the connection determination logic, etc.). As an example, if user devices identifies each of the medical devices or disposables needed to generate a preferred architecture connection for each IV line in the image, user device 102 may determine the connections between the pair of medical devices by giving more probability for preferred connections including pairs of medical devices that are connected in the preferred architecture (e.g., a lumen adaptor connected to IV tubing, etc.). For example, a proximity or distance parameter of a pair of preferred architecture pieces may be weighted more strongly compared to other parameters (e.g., orientation, collinearity, etc.) when the medical devices are determined to be within a threshold proximity or distance of each other. In some non-limiting embodiments or aspects, in response to determining an unlikely parameter associated with a pair of medical devices (e.g., a dressing being a threshold distance, such as 30 cm, away from a lumen adapter, etc.) user device 102 may prompt a user to retake the image and/or provide a notification to the user of the unlikely parameters (e.g., asking if there are multiple catheter components in a single image, etc.).

In some non-limiting embodiments or aspects, user device 102 may adjust a weight used to determine whether the pair of medical devices are connected to each other based on a determination that a medical device of the pair of medical devices is connected to another medical device (e.g., another medical device in a preferred IV line architecture including the pair of medical devices). For example, for a pair of medical devices including a first medical device and a second medical device, if user device determines that the first medical device is already connected to a third medical device of a preferred IV line structure including the first medical device, the second medical device, and the third medical device, user device 102 may give a higher probability of the first medical device and the second medical device being connected because such a connection complete the preferred IV line architecture.

In such an example, if user device 102 identifies only a portion of the medical devices or disposables needed to generate a preferred architecture connection for each IV line in the image, user device 102 may determine the connections between the pair of medical devices by giving more probability for preferred connections including pairs of medical devices that are connected in the preferred architecture and are of a predetermined type of medical device (e.g., devices that are not caps, etc.). For example, if a triple lumen catheter is expected for a preferred architecture and only a single IV tubing is identified in the image, user device 102 may automatically expect caps on the other lumens or lumen adapters, and if user device 102 does not identify the caps in the image, user device 102 may prompt the user to retake the image and/or provide a notification to the user that requests clarification.

In such an example, user device 102 may determine, based on the type of each medical device of the plurality of medical devices, that no medical devices of the plurality of medical devices are associated with a preferred IV line architecture. For example, in response to determining that no medical devices of the plurality of medical devices are associated with the preferred IV line architecture, user device 102 may prompt a user to obtain another image.

In such an example, user device 102 may determine, based on the type of each medical device of the plurality of medical devices, that a number of medical devices of the plurality of medical devices is greater than a number of medical devices associated with a preferred IV line architecture. For example, if a number of medical devices identified is greater than a number needed to generate preferred architecture connections for each lumen or IV line, it implies that there may be daisy-chaining or multiple tagged components inside the image. As an example, if user device 102 identifies more IV tubing sets than catheter lumens, user device 102 may give more weight to multiple tubing to IV tubing Y-port connections in the probability-based tree building logic. As another example, if user device 102 identifies a threshold number of caps, user device 102 may give more weight to the proximity parameter for detecting a distance of medical devices from open ports.

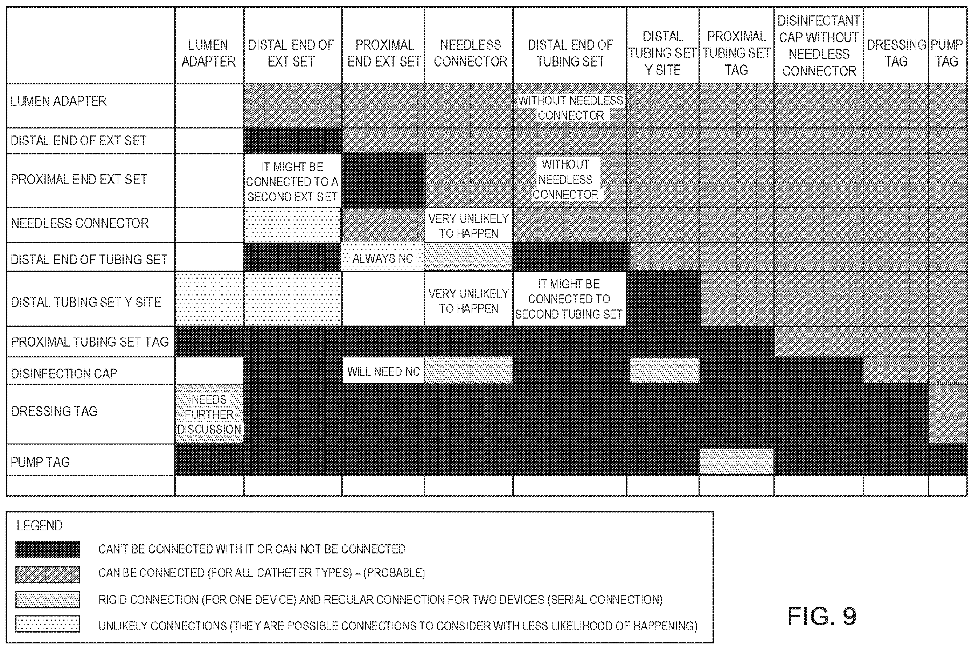

Referring now to FIG. 9, FIG. 9 is a chart of example unlikely but possible connections between medical devices. For example, a probability of connection for medical devices of a same class or type may be zero (a tube may be an exception - however a tube may have a secondary tube Y-port tag). For example, unlikely connections between medical devices 108 may be connections between medical devices that are unexpected and/or serve no purpose; however, such connections may not be incorrect and/or may potentially be initiated by some users. Loose dressings and/or fiducial markers 110 for dressings may be left around a catheter insertion site and appear close to a tubing set or other identified medical devices, and user device 102 may adjust a weight or give priority to dressing tag proximity or distance from catheter lumen adapters and the tag vectors angle from each other in the probability-based tree building logic to determine that these loose fiducial markers or tags are not attached to any of the medical devices. Loose caps may float around on a bed or a patient close to other tagged objects, and user device 102 may automatically determine that a cap is not connected to another medical device if a distance between the cap and the other medical device satisfies a predetermined threshold distance. Loose Tubing sets on the bed or patient may be processed in a same or similar manner as loose caps. It is unlikely but possible that lumen adapters may be connected to each other, and user device 102 may determine whether lumen adapters are connected to each other based on a distance and/or a collinearity of the two adapters.

In some non-limiting embodiments or aspects, user device 102 may process the position information and/or the types of the plurality of medical devices 108 with a machine learning model to determine probabilities that pairs of medical devices are connected. For example, user device 102 may generate a prediction model (e.g., an estimator, a classifier, a prediction model, a detector model, etc.) using machine learning techniques including, for example, supervised and/or unsupervised techniques, such as decision trees (e.g., gradient boosted decision trees, random forests, etc.), logistic regressions, artificial neural networks (e.g., convolutional neural networks, etc.), Bayesian statistics, learning automata, Hidden Markov Modeling, linear classifiers, quadratic classifiers, association rule learning, and/or the like. The prediction machine learning model may be trained to provide an output including a prediction of whether a pair of medical devices are connected to each other. In such an example, the prediction may include a probability (e.g., a likelihood, etc.) that the pair of medical devices are connected to each other.

User device 102 may generate the prediction model based on position information associated with each medical device and/or types of each medical device (e.g., training data, etc.). In some implementations, the prediction model is designed to receive, as an input, the position information associated with each medical device in a pair of medical devices (e.g., a proximity between the devices, an orientation between the devices, a collinearity between the devices, etc.) and provide, as an output, a prediction (e.g., a probability, a likelihood, a binary output, a yes-no output, a score, a prediction score, a classification, etc.) as to whether the pair of medical devices are connected to each other. In some non-limiting embodiments or aspects, user device 102 stores the prediction model (e.g., stores the model for later use). In some non-limiting embodiments or aspects, user device 102 stores the initial prediction model in a data structure (e.g., a database, a linked list, a tree, etc.). In some non-limiting embodiments, the data structure is located within user device 102 or external (e.g., remote from) user device 102 (e.g., within management system 104, etc.).

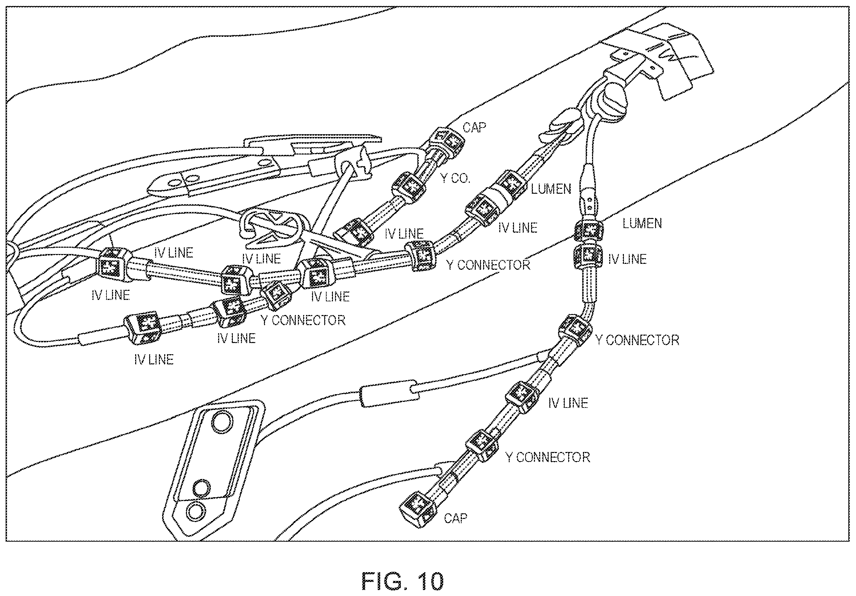

As shown in FIG. 4, at step 408, process 400 includes generating a representation of at least one IV line. For example, user device 102 may generate, based on the pairs of medical devices that are determined to be connected to each other, a representation of at least one IV line including the pairs of medical devices that are determined to be connected to each other. As an example, and referring also to FIG. 10, which illustrates an implementation of non-limiting embodiments or aspects of a representation of IV lines (e.g., a catheter tree, etc.), user device 102 may automatically draw lines connecting medical devices (e.g., catheter tree components, etc.) individually for each IV line and/or display identifying information associated with each IV line and/or individual medical devices 108 in each IV line. In such an example, user device 102 may automatically draw and display the lines on an image and/or within a series of images, such as a live video feed, and/or the like of the medical devices/catheter insertion site. For example, user device 102 may generate a digital representation of each IV line including each pair of medical devices in each IV line according to the pairs of medical devices that are determined to be connected to each other. In such an example, user device 102 may associate each IV line with a fluid source or pump of an infusion pump and monitor a flow of a fluid in the IV line(s) based at least partially on the representation of the fluid flow path. As an example, user device 102 may control an audio and/or visual output device to output an audible and/or visible indication, wherein the audible and/or visible indication indicates a status of the IV line and/or the fluid flowing therethrough. For example, user device 102 may generate a catheter tree or a logical IV branch structure that maps over the physical IV branch structure and includes a unique node identifier for each medical device of the physical IV branch structure, each connector or entry/exit point to a fluid flow path formed by the medical devices, and/or each element of a medical device associated with an action that can affect the fluid flow path, *(e.g.,* a valve in a medical device).

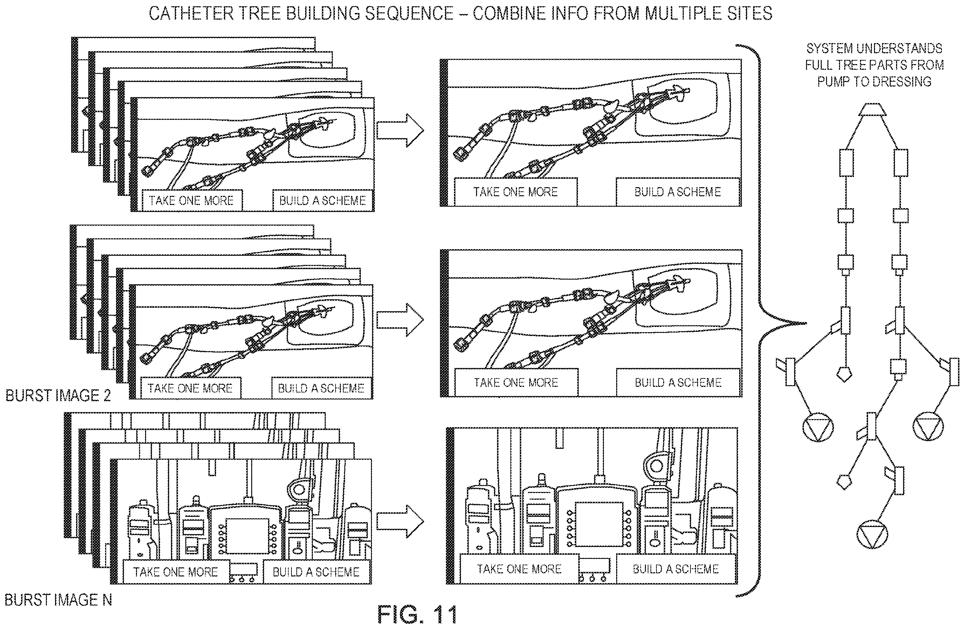

In some non-limiting embodiments or aspects, an image capture device of user device 102 may capture a plurality of images from a plurality of different fields of view or locations. For example, and referring also to FIG. 11, which illustrates an example catheter tree building sequence, an image capture device of user device 102 may capture, using a burst capture technique, a series of images from a plurality of different field of views or locations (e.g., a first field of view or location including an insertion site on a patient, a second field of view or location including an infusion pump, etc.). A series of images may include tagged and/or untagged medical devices 108. User device 102 may continuously integrate and/or combine position information associated with medical devices 108 determined from each image in a series of images and from each series of images captured from each of the field of view or location and use the integrated position information to determine pairs of the medical devices 108 that are connected to each other and to build a catheter tree including IV lines formed by the pairs of medical devices that are determined to be connected to each other (e.g., a catheter tree including medical devices from an insertion site and/or dressing tag to an infusion pump or module, etc.).

In some non-limiting embodiments or aspects, user device 102 may compare medication, medication dosage, medication delivery route or IV line, and/or medication delivery time associated with an IV line to an approved patient, approved medication, approved medication dosage, approved medication delivery route or IV line, and/or approved medication delivery time associated with the patient identifier and/or a medication identifier to reduce medication administration errors. User device 102 may issue an alert and/or the infusion pump to stop fluid flow and/or adjust fluid flow based on a current representation of the at least one IV line (e.g., based on a current state of the catheter tree, etc.). For example, if a medication scheduled for or loaded into the infusion pump at a point of entry in the fluid flow path is determined to be an improper medication for the patient, an improper dosage for the patient and/or medication, an improper medication delivery route for the patient and/or medication (e.g., improper point of entry to the fluid flow path), and/or an improper medication delivery time for the patient and/or medication, user device 102 may issue an alert and/or control the infusion pump to stop or prevent the fluid flow.

In some non-limiting embodiments or aspects, user device 102 may determine a dwell time of medical devices 108 (e.g., in environment 100, etc.) and/or connections thereof (e.g., an amount or duration of time a medical device is connected to another medical device and/or the patient, etc.). For example, user device 102 may determine, based on the probabilities that pairs of medical devices are connected, a time at which a medical device 108 enters the environment 100 and/or is connected to another medical device and/or the patient. As an example, user device 102 may automatically determine and/or record, based on the pairs of medical devices that are determined to be connected to each other (e.g., over a period of time, over a series of images, over a live video feed, etc.), a time at which a medical device 108 is connected to another medical device and/or the patient, a duration of time form the connection time to a current time that the medical device 108 has been connected thereto, and/or a time at which the medical device is disconnected from another medical device and/or the patient.

In some non-limiting embodiments or aspects, user device 102 may automatically determine and/or record, based on the pairs of medical devices that are determined to be connected to each other (e.g., over a period of time, over a series of images, over a live video feed, etc.), a frequency at which a medical device 108 is connected to another medical device or a particular type of medical device. For example, user device 102 may determine a frequency at which one or more disinfecting caps are connected to an IV access port, a luer tip, and/or the like and/or a duration that each cap is connected thereto.

In some non-limiting embodiments or aspects, user device 102 may compare a dwell time and/or a connection frequency associated with a medical device to a dwell time threshold and/or a frequency threshold associated with the medical device and/or a connection including the medical devices, and, if the dwell time and/or the connection frequency satisfies the dwell time threshold and/or the frequency threshold, provide an alert (e.g., via user device 102, etc.) associated therewith. For example, user device 102 may provide an alert indicating that it is time to replace a medical device in the catheter tree with a new medical device and/or that a medical device should be disinfected and/or flushed.

Although embodiments or aspects have been described in detail for the purpose of illustration and description, it is to be understood that such detail is solely for that purpose and that embodiments or aspects are not limited to the disclosed embodiments or aspects, but, on the contrary, are intended to cover modifications and equivalent arrangements that are within the scope of the appended claims. For example, it is to be understood that the present disclosure contemplates that, to the extent possible, one or more features of any embodiment or aspect can be combined with one or more features of any other embodiment or aspect. In fact, many of these features can be combined in ways not specifically recited in the claims and/or disclosed in the specification. Although each dependent claim listed below may directly depend on only one claim, the disclosure of possible implementations includes each dependent claim in combination with every other claim in the claim set.

## Claims

1. A system, comprising:
at least one processor programmed and/or configured to:
obtain an image of a plurality of medical devices, captured by an image capture device;
determine, based on the image, position information associated with a three-dimensional , 3D, position of the plurality of medical devices relative to the image capture device;
determine, based on the position information, pairs of medical devices of the plurality of medical devices that are connected to each other; and
generate, based on the pairs of medical devices that are determined to be connected to each other, a representation of at least one intravenous, IV, line including the pairs of medical devices that are determined to be connected to each other.

2. The system of claim 1, wherein the at least one processor is programmed and/or configured to determine the pairs of medical devices that are connected to each other by:
for each pair of medical devices of the plurality of medical devices, determining, based on the position information associated with that pair of medical devices, a probability that that pair of medical devices are connected to each other.

3. The system of claim 2, wherein the at least one processor is programmed and/or configured to determine the pairs of medical devices that are connected to each other by:
for each pair of medical devices of the plurality of medical devices, determining, based on the position information associated with that pair of medical devices, the following parameters: a distance between center points associated with that pair of medical devices, an angular difference between orientations of that pair medical devices, and an angle from collinearity of that pair of medical devices, wherein the probability that that pair of medical devices is connected is determined based on the distance between the center points of that pair of medical devices, the angular difference between the orientations of that pair of medical devices, and the angle from collinearity of that pair of medical devices.

4. The system of claim 2, wherein, for each medical device of the plurality of medical devices, that medical device is determined to be connected to another medical device in a pair of medical devices including that medical device that is associated with a highest probability of the pairs of medical devices including that medical device.

5. The system of claim 2, wherein the at least one processor is programmed and/or configured to determine the position information associated with the 3D position of the plurality of medical devices relative to the image capture device by:
determining, based on the image, a type of each medical device of the plurality of medical devices, wherein, for a pair of medical devices of the plurality of medical devices, the probability that that pair of medical devices are connected to each other is further determined based on the type of each medical device in that pair of medical devices.

6. The system of claim 5, wherein the at least one processor is programmed and/or configured to determine the pairs of medical devices that are connected to each other by:
identifying, based on the type of each medical device of the plurality of medical devices, a set of medical devices of the plurality of medical devices that are associated with a preferred IV line architecture; and
for a pair of medical devices included in the set of medical devices associated with the preferred IV line architecture, adjusting a weight used to determine whether the pair of medical devices are connected to each other.

7. The system of claim 5, wherein the at least one processor is programmed and/or configured to determine the pairs of medical devices that are connected to each other by:
determining, based on the type of each medical device of the plurality of medical devices, that no medical devices of the plurality of medical devices are associated with a preferred IV line architecture; and
in response to determining that no medical devices of the plurality of medical devices are associated with the preferred IV line architecture, prompting a user to obtain another image.

8. The system of claim 1, wherein a first group of medical devices of the plurality of medical devices is associated with a plurality of fiducial markers, wherein the plurality of fiducial markers encapsulates a plurality of identifiers associated with the first group of medical devices and pose information associated with the 3D position of the plurality of fiducial markers, and wherein the at least one processor is programmed and/or configured to determine the position information associated with the 3D position of the plurality of medical devices relative to the image capture device by:
determining, based on the image, the plurality of identifiers associated with the first group of medical devices and the pose information associated with the 3D position of the plurality of fiducial markers,
wherein, for each medical device of the first group of medical devices, the position information associated with the 3D position of that medical device relative to the image capture device is determined as the 3D position of a fiducial marker associated with that medical device relative to the image capture device.

9. The system of claim 8, wherein the plurality of fiducial markers includes a plurality of AprilTags; or
wherein, for each medical device of the first group of medical devices, the 3D position of the fiducial marker associated with that medical device relative to the image capture device includes x, y, and z coordinates of the fiducial marker and directional vectors for Z, Y, and X axes of the fiducial marker; or
wherein the plurality of identifiers is associated with a plurality of types of medical devices; or
wherein the plurality of identifiers includes a plurality of unique identifiers; or
wherein a second group of medical devices of the plurality of medical devices is not associated with a fiducial marker, and wherein the at least one processor is programmed and/or configured to determine the position information associated with the 3D position of the plurality of medical devices relative to the image capture device by:
for each medical device of the second group of medical devices, determining, based on the image, using an object recognition technique, a type of that medical device and the 3D position of that medical device relative to the image capture device; or
wherein the plurality of fiducial markers is rigidly affixed to rigid portions of the first group of medical devices such that the plurality of fiducial markers cannot translate along and rotate around the first group of medical devices.

10. The system of claim 1, wherein the at least one processor is programmed and/or configured to obtain the image of the plurality of medical devices further by:
capturing, with the image capture device, the image of the plurality of medical devices.

11. The system of claim 10, wherein the image includes a series of images, wherein preferably the image capture device captures the series of images using a burst capture technique, or wherein the image capture device includes a stereo camera, and wherein the position information associated with the 3D position of the plurality of medical devices relative to the image capture device is determined using a Structure from Motion , SfM, algorithm, or wherein the image capture device includes a LiDAR system, and wherein the image includes a LiDAR point cloud.

12. The system of claim 1, wherein the at least one processor is programmed and/or configured to generate the representation of the at least one IV line by automatically displaying, on the image, lines connecting the pairs of medical devices that are determined to be connected to each other in the at least one IV line.

13. The system of claim 1, wherein the at least one IV line is associated with at least one pump of an infusion pump in the representation.

14. The system of claim 1, wherein the at least one processor is programmed and/or configured to:
determine, based on the pairs of medical devices that are determined to be connected to each other, a dwell time indicating a duration of time a medical device of the plurality of medical devices is connected to another medical device of the plurality of medical devices; and
in response to the dwell time satisfying a threshold dwell time, provide, via a user device, an alert associated with the medical device.

15. A method, comprising:
obtaining, with at least one processor, an image of a plurality of medical devices, captured by an image capture device;
determining, with the at least one processor, based on the image, position information associated with a three-dimensional , 3D, position of the plurality of medical devices relative to the image capture device;
determining, with the at least one processor, based on the position information, pairs of medical devices of the plurality of medical devices that are connected to each other; and
generating, with the at least one processor, based on the pairs of medical devices that are determined to be connected to each other, a representation of at least one intravenous, IV, line including the pairs of medical devices that are determined to be connected to each other.

16. The method of claim 15, wherein determining the pairs of medical devices that are connected to each other further includes:
for each pair of medical devices of the plurality of medical devices, determining, based on the position information associated with that pair of medical devices, a probability that that pair of medical devices are connected to each other.

17. The method of claim 16, wherein determining the pairs of medical devices that are connected to each other further includes:
for each pair of medical devices of the plurality of medical devices, determining, based on the position information associated with that pair of medical devices, the following parameters: a distance between center points associated with that pair of medical devices, an angular difference between orientations of that pair medical devices, and an angle from collinearity of that pair of medical devices, wherein the probability that that pair of medical devices is connected is determined based on the distance between the center points of that pair of medical devices, the angular difference between the orientations of that pair of medical devices, and the angle from collinearity of that pair of medical devices.

18. The method of claim 16, wherein, for each medical device of the plurality of medical devices, that medical device is determined to be connected to another medical device in a pair of medical devices including that medical device that is associated with a highest probability of the pairs of medical devices including that medical device.

19. The method of claim 16, wherein determining the position information associated with the 3D position of the plurality of medical devices relative to the image capture device further includes:
determining, based on the image, a type of each medical device of the plurality of medical devices, wherein, for a pair of medical devices of the plurality of medical devices, the probability that that pair of medical devices are connected to each other is further determined based on the type of each medical device in that pair of medical devices.

20. The method of claim 19, wherein determining the pairs of medical devices that are connected to each other further includes:
identifying, based on the type of each medical device of the plurality of medical devices, a set of medical devices of the plurality of medical devices that are associated with a preferred IV line architecture; and
for a pair of medical devices included in the set of medical devices associated with the preferred IV line architecture, adjusting a weight used to determine whether the pair of medical devices are connected to each other.

21. The method of claim 16, wherein determining the pairs of medical devices that are connected to each other further includes:
determining, based on the type of each medical device of the plurality of medical devices, that no medical devices of the plurality of medical devices are associated with a preferred IV line architecture; and
in response to determining that no medical devices of the plurality of medical devices are associated with the preferred IV line architecture, prompting a user to obtain another image.

22. The method of claim 15, wherein a first group of medical devices of the plurality of medical devices is associated with a plurality of fiducial markers, wherein the plurality of fiducial markers encapsulates a plurality of identifiers associated with the first group of medical devices and pose information associated with the 3D position of the plurality of fiducial markers, and wherein determining the position information associated with the 3D position of the plurality of medical devices relative to the image capture device includes:
determining, based on the image, the plurality of identifiers associated with the first group of medical devices and the pose information associated with the 3D position of the plurality of fiducial markers,
wherein, for each medical device of the first group of medical devices, the position information associated with the 3D position of that medical device relative to the image capture device is determined as the 3D position of a fiducial marker associated with that medical device relative to the image capture device.

23. The method of claim 22, wherein the plurality of fiducial markers includes a plurality of AprilTags; or
wherein, for each medical device of the first group of medical devices, the 3D position of the fiducial marker associated with that medical device relative to the image capture device includes x, y, and z coordinates of the fiducial marker and directional vectors for Z, Y, and X axes of the fiducial marker; or
wherein the plurality of identifiers is associated with a plurality of types of medical devices; or
wherein the plurality of identifiers includes a plurality of unique identifiers; or
wherein a second group of medical devices of the plurality of medical devices is not associated with a fiducial marker, and wherein determining the position information associated with the 3D position of the plurality of medical devices relative to the image capture device further includes:
for each medical device of the second group of medical devices, determining, based on the image, using an object recognition technique, a type of that medical device and the 3D position of that medical device relative to the image capture device; or
wherein the plurality of fiducial markers is rigidly affixed to rigid portions of the first group of medical devices such that the plurality of fiducial markers cannot translate along and rotate around the first group of medical devices.

24. The method of claim 15, wherein obtaining the image of the plurality of medical devices further includes:
capturing, with the image capture device, the image of the plurality of medical devices.

25. The method of claim 24, wherein the image includes a series of images, wherein preferably the image capture device captures the series of images using a burst capture technique, or wherein the image capture device includes a stereo camera, and wherein the position information associated with the 3D position of the plurality of medical devices relative to the image capture device is determined using a Structure from Motion , SfM, algorithm, or wherein the image capture device includes a LiDAR system, and wherein the image includes a LiDAR point cloud.

26. The method of claim 15, wherein generating the representation of the at least one IV line includes automatically displaying, on the image, lines connecting the pairs of medical devices that are determined to be connected to each other in the at least one IV line.

27. The method of claim 15, wherein the at least one IV line is associated with at least one pump of an infusion pump in the representation.

28. The method of claim 15, further comprising:
determining, with the at least one processor, based on the pairs of medical devices that are determined to be connected to each other, a dwell time indicating a duration of time a medical device of the plurality of medical devices is connected to another medical device of the plurality of medical devices; and
in response to the dwell time satisfying a threshold dwell time, providing, with the at least one processor, via a user device, an alert associated with the medical device.

## Patentansprüche

1. System, das aufweist:
mindestens einen Prozessor, der für Folgendes programmiert und/oder ausgebildet ist:
Erfassen eines Bildes einer Vielzahl medizinischer Vorrichtungen, das von einer Bildaufnahmevorrichtung aufgenommen wird;
Bestimmen, auf der Grundlage des Bildes, von Positionsinformationen, die einer dreidimensionalen, 3D, Position der Vielzahl medizinischer Vorrichtungen relativ zur Bildaufnahmevorrichtung zugeordnet sind;
Bestimmen, auf der Grundlage der Positionsinformationen, von Paaren medizinischer Vorrichtungen aus der Vielzahl medizinischer Vorrichtungen, die miteinander verbunden sind; und
Erzeugen, auf der Grundlage der Paare medizinischer Vorrichtungen, die als miteinander verbunden bestimmt werden, einer Darstellung mindestens einer intravenösen, IV, Leitung, welche die Paare medizinischer Vorrichtungen umfasst, die als miteinander verbunden bestimmt werden.

2. System nach Anspruch 1, wobei der mindestens eine Prozessor so programmiert und/oder ausgebildet ist, dass er die Paare medizinischer Vorrichtungen bestimmt, die miteinander verbunden sind, indem:
für jedes Paar medizinischer Vorrichtungen aus der Vielzahl medizinischer Vorrichtungen auf der Grundlage der diesem Paar medizinischer Vorrichtungen zugeordneten Positionsinformationen eine Wahrscheinlichkeit bestimmt wird, dass dieses Paar medizinischer Vorrichtungen miteinander verbunden ist.

3. System nach Anspruch 2, wobei der mindestens eine Prozessor so programmiert und/oder ausgebildet ist, dass er die Paare medizinischer Vorrichtungen bestimmt, die miteinander verbunden sind, indem:
für jedes Paar medizinischer Vorrichtungen aus der Vielzahl medizinischer Vorrichtungen auf der Grundlage der diesem Paar medizinischer Vorrichtungen zugeordneten Positionsinformationen die folgenden Parameter bestimmt werden: ein Abstand zwischen den diesem Paar medizinischer Vorrichtungen zugeordneten Mittelpunkten, ein Winkelunterschied zwischen den Ausrichtungen dieses Paares medizinischer Vorrichtungen, und ein Winkel der Abweichung von der Kollinearität dieses Paares medizinischer Vorrichtungen, wobei die Wahrscheinlichkeit, dass dieses Paar medizinischer Vorrichtungen miteinander verbunden ist, auf der Grundlage des Abstands zwischen den Mittelpunkten dieses Paares medizinischer Vorrichtungen, des Winkelunterschieds zwischen den Ausrichtungen dieses Paares medizinischer Vorrichtungen, und des Winkels der Abweichung von der Kollinearität dieses Paares medizinischer Vorrichtungen bestimmt wird.

4. System nach Anspruch 2, wobei für jede medizinische Vorrichtung aus der Vielzahl medizinischer Vorrichtungen bestimmt wird, dass diese medizinische Vorrichtung mit einer anderen medizinischen Vorrichtung in einem Paar medizinischer Vorrichtungen verbunden ist, das diese medizinische Vorrichtung umfasst und dem unter den Paaren medizinischer Vorrichtungen, die diese medizinische Vorrichtung umfassen, die höchste Wahrscheinlichkeit zugeordnet ist.

5. System nach Anspruch 2, wobei der mindestens eine Prozessor so programmiert und/oder ausgebildet ist, dass er die Positionsinformationen bestimmt, die der 3D-Position der Vielzahl medizinischer Vorrichtungen relativ zur Bildaufnahmevorrichtung zugeordnet sind, durch:
Bestimmen eines Typs jeder medizinischen Vorrichtung aus der Vielzahl medizinischer Vorrichtungen auf der Grundlage des Bildes, wobei für ein Paar medizinischer Vorrichtungen aus der Vielzahl medizinischer Vorrichtungen die Wahrscheinlichkeit, dass dieses Paar medizinischer Vorrichtungen miteinander verbunden ist, zusätzlich auf der Grundlage des Typs jeder medizinischen Vorrichtung in diesem Paar medizinischer Vorrichtungen bestimmt wird.

6. System nach Anspruch 5, wobei der mindestens eine Prozessor so programmiert und/oder ausgebildet ist, dass er die Paare medizinischer Vorrichtungen bestimmt, die miteinander verbunden sind, durch:
Identifizieren, auf der Grundlage des Typs jeder medizinischen Vorrichtung der Vielzahl medizinischer Vorrichtungen, eines Satzes medizinischer Vorrichtungen aus der Vielzahl medizinischer Vorrichtungen, die einer bevorzugten IV-Leitungsarchitektur zugeordnet sind; und
für ein Paar medizinischer Vorrichtungen, das in dem Satz medizinischer Vorrichtungen enthalten ist, welcher der bevorzugten IV-Leitungsarchitektur zugeordnet ist, Anpassen eines Gewichts, das verwendet wird, um zu bestimmen, ob das Paar medizinischer Vorrichtungen miteinander verbunden ist.

7. System nach Anspruch 5, wobei der mindestens eine Prozessor so programmiert und/oder ausgebildet ist, dass er die Paare medizinischer Vorrichtungen bestimmt, die miteinander verbunden sind, durch:
Bestimmen, auf der Grundlage des Typs jeder medizinischen Vorrichtung der Vielzahl medizinischer Vorrichtungen, dass keine medizinischen Vorrichtungen der Vielzahl medizinischer Vorrichtungen einer bevorzugten IV-Leitungsarchitektur zugeordnet sind; und
als Reaktion auf das Bestimmen, dass keine medizinischen Vorrichtungen der Vielzahl medizinischer Vorrichtungen der bevorzugten IV-Leitungsarchitektur zugeordnet sind, Auffordern eines Benutzers, ein weiteres Bild zu erfassen.

8. System nach Anspruch 1, wobei eine erste Gruppe der Vielzahl medizinischer Vorrichtungen einer Vielzahl von Referenzmarken zugeordnet ist, wobei die Vielzahl von Referenzmarken eine Vielzahl von Kennungen, die der ersten Gruppe medizinischer Vorrichtungen zugeordnet sind, sowie Lageinformationen, die der 3D-Position der Vielzahl von Referenzmarken zugeordnet sind, enthält, und wobei der mindestens eine Prozessor so programmiert und/oder ausgebildet ist, dass er die Positionsinformationen, die der 3D-Position der Vielzahl medizinischer Vorrichtungen relativ zur Bildaufnahmevorrichtung zugeordnet sind, bestimmt durch:
Bestimmen der Vielzahl von Kennungen, die der ersten Gruppe medizinischer Vorrichtungen zugeordnet sind, sowie der Lageinformationen, die der 3D-Position der Vielzahl von Referenzmarken zugeordnet sind, auf der Grundlage des Bildes,
wobei für jede medizinische Vorrichtung der ersten Gruppe medizinischer Vorrichtungen die Positionsinformationen, die der 3D-Position dieser medizinischen Vorrichtung relativ zur Bildaufnahmevorrichtung zugeordnet sind, als die 3D-Position einer dieser medizinischen Vorrichtung zugeordneten Referenzmarke relativ zur Bildaufnahmevorrichtung bestimmt werden.

9. System nach Anspruch 8, wobei die Vielzahl von Referenzmarken eine Vielzahl von AprilTags umfasst; oder
wobei für jede medizinische Vorrichtung der ersten Gruppe medizinischer Vorrichtungen die 3D-Position der dieser medizinischen Vorrichtung zugeordneten Referenzmarke relativ zur Bildaufnahmevorrichtung die x-, y- und z-Koordinaten der Referenzmarke sowie Richtungsvektoren für die Z-, Y- und X-Achsen der Referenzmarke umfasst; oder
wobei die Vielzahl von Kennungen einer Vielzahl von Typen medizinischer Vorrichtungen zugeordnet ist; oder
wobei die Vielzahl von Kennungen eine Vielzahl von eindeutigen Kennungen umfasst; oder
wobei eine zweite Gruppe medizinischer Vorrichtungen der Vielzahl medizinischer Vorrichtungen nicht einer Referenzmarke zugeordnet ist, und wobei der mindestens eine Prozessor so programmiert und/oder ausgebildet ist, dass er die Positionsinformationen bestimmt, die der 3D-Position der Vielzahl medizinischer Vorrichtungen relativ zur Bildaufnahmevorrichtung zugeordnet sind, durch:
für jede medizinische Vorrichtung der zweiten Gruppe medizinischer Vorrichtungen, Bestimmen eines Typs dieser medizinischen Vorrichtung und der 3D-Position dieser medizinischen Vorrichtung relativ zur Bildaufnahmevorrichtung auf der Grundlage des Bildes unter Verwendung einer Objekterkennungstechnik; oder
wobei die Vielzahl von Referenzmarken fest an starren Abschnitten der ersten Gruppe medizinischer Vorrichtungen befestigt ist, sodass sich die Vielzahl von Referenzmarken weder entlang der ersten Gruppe medizinischer Vorrichtungen verschieben noch um diese drehen kann.

10. System nach Anspruch 1, wobei der mindestens eine Prozessor so programmiert und/oder ausgebildet ist, dass er das Bild der Vielzahl medizinischer Vorrichtungen ferner erfasst durch:
Aufnehmen des Bildes der Vielzahl medizinischer Vorrichtungen mit der Bildaufnahmevorrichtung.

11. System nach Anspruch 10, wobei das Bild eine Bildserie umfasst, wobei die Bildaufnahmevorrichtung die Bildserie vorzugsweise unter Verwendung einer Serienaufnahmetechnik aufnimmt, oder wobei die Bildaufnahmevorrichtung eine Stereokamera aufweist, und wobei die Positionsinformationen, die der 3D-Position der Vielzahl medizinischer Vorrichtungen relativ zur Bildaufnahmevorrichtung zugeordnet sind, unter Verwendung eines Structure-from-Motion-, SfM, Algorithmus bestimmt werden, oder wobei die Bildaufnahmevorrichtung ein LiDAR-System aufweist, und wobei das Bild eine LiDAR-Punktwolke aufweist.

12. System nach Anspruch 1, wobei der mindestens eine Prozessor so programmiert und/oder ausgebildet ist, dass er die Darstellung der mindestens einen IV-Leitung erzeugt, indem er auf dem Bild automatisch Linien anzeigt, welche die Paare medizinischer Vorrichtungen verbinden, die als in der mindestens einen IV-Leitung miteinander verbunden bestimmt werden.

13. System nach Anspruch 1, wobei die mindestens eine IV-Leitung in der Darstellung mindestens einer Pumpe einer Infusionspumpe zugeordnet ist.

14. System nach Anspruch 1, wobei der mindestens eine Prozessor für Folgendes programmiert und/oder ausgebildet ist:
Bestimmen, auf der Grundlage der Paare medizinischer Vorrichtungen, bei denen bestimmt wird, dass sie miteinander verbunden sind, einer Verweildauer, die angibt, wie lange eine medizinische Vorrichtung aus der Vielzahl medizinischer Vorrichtungen mit einer anderen medizinischen Vorrichtung aus der Vielzahl medizinischer Vorrichtungen verbunden ist; und
als Reaktion darauf, dass die Verweildauer einen Verweildauerschwellenwert erfüllt, Ausgeben einer der medizinischen Vorrichtung zugeordneten Warnmeldung über eine Benutzervorrichtung.

15. Verfahren, das umfasst:
Erfassen, mittels mindestens eines Prozessors, eines Bildes einer Vielzahl medizinischer Vorrichtungen, das von einer Bildaufnahmevorrichtung aufgenommen wird;
Bestimmen, mittels des mindestens einen Prozessors, auf der Grundlage des Bildes, von Positionsinformationen, die einer dreidimensionalen, 3D, Position der Vielzahl medizinischer Vorrichtungen relativ zur Bildaufnahmevorrichtung zugeordnet sind;
Bestimmen, mittels des mindestens einen Prozessors, auf der Grundlage der Positionsinformationen, von Paaren medizinischer Vorrichtungen aus der Vielzahl medizinischer Vorrichtungen, die miteinander verbunden sind; und
Erzeugen, mittels des mindestens einen Prozessors, auf der Grundlage der Paare medizinischer Vorrichtungen, die als miteinander verbunden bestimmt werden, einer Darstellung mindestens einer intravenösen, IV, Leitung, welche die Paare medizinischer Vorrichtungen umfasst, die als miteinander verbunden bestimmt werden.

16. Verfahren nach Anspruch 15, wobei das Bestimmen der Paare medizinischer Vorrichtungen, die miteinander verbunden sind, ferner umfasst:
für jedes Paar medizinischer Vorrichtungen aus der Vielzahl medizinischer Vorrichtungen, Bestimmen, auf der Grundlage der diesem Paar medizinischer Vorrichtungen zugeordneten Positionsinformationen, einer Wahrscheinlichkeit, dass dieses Paar medizinischer Vorrichtungen miteinander verbunden ist.

17. Verfahren nach Anspruch 16, wobei das Bestimmen der Paare medizinischer Vorrichtungen, die miteinander verbunden sind, ferner umfasst:
für jedes Paar medizinischer Vorrichtungen aus der Vielzahl medizinischer Vorrichtungen, Bestimmen, auf der Grundlage der diesem Paar medizinischer Vorrichtungen zugeordneten Positionsinformationen, der folgenden Parameter: eines Abstands zwischen den diesem Paar medizinischer Vorrichtungen zugeordneten Mittelpunkten, eines Winkelunterschieds zwischen den Ausrichtungen dieses Paares medizinischer Vorrichtungen, und eines Winkels der Abweichung von der Kollinearität dieses Paares medizinischer Vorrichtungen, wobei die Wahrscheinlichkeit, dass dieses Paar medizinischer Vorrichtungen miteinander verbunden ist, auf der Grundlage des Abstands zwischen den Mittelpunkten dieses Paares medizinischer Vorrichtungen, des Winkelunterschieds zwischen den Ausrichtungen dieses Paares medizinischer Vorrichtungen, und des Winkels der Abweichung von der Kollinearität dieses Paares medizinischer Vorrichtungen bestimmt wird.

18. Verfahren nach Anspruch 16, wobei für jede medizinische Vorrichtung aus der Vielzahl medizinischer Vorrichtungen bestimmt wird, dass diese medizinische Vorrichtung mit einer anderen medizinischen Vorrichtung in einem Paar medizinischer Vorrichtungen verbunden ist, das diese medizinische Vorrichtung umfasst und dem unter den Paaren medizinischer Vorrichtungen, die diese medizinische Vorrichtung umfassen, die höchste Wahrscheinlichkeit zugeordnet ist.

19. Verfahren nach Anspruch 16, wobei das Bestimmen der Positionsinformationen, die der 3D-Position der Vielzahl medizinischer Vorrichtungen relativ zur Bildaufnahmevorrichtung zugeordnet sind, ferner umfasst:
Bestimmen eines Typs jeder medizinischen Vorrichtung aus der Vielzahl medizinischer Vorrichtungen auf der Grundlage des Bildes, wobei für ein Paar medizinischer Vorrichtungen aus der Vielzahl medizinischer Vorrichtungen die Wahrscheinlichkeit, dass dieses Paar medizinischer Vorrichtungen miteinander verbunden ist, zusätzlich auf der Grundlage des Typs jeder medizinischen Vorrichtung in diesem Paar medizinischer Vorrichtungen bestimmt wird.

20. Verfahren nach Anspruch 19, wobei das Bestimmen der Paare medizinischer Vorrichtungen, die miteinander verbunden sind, ferner umfasst:
Identifizieren, auf der Grundlage des Typs jeder medizinischen Vorrichtung der Vielzahl medizinischer Vorrichtungen, eines Satzes medizinischer Vorrichtungen aus der Vielzahl medizinischer Vorrichtungen, die einer bevorzugten IV-Leitungsarchitektur zugeordnet sind; und
für ein Paar medizinischer Vorrichtungen, das in dem Satz medizinischer Vorrichtungen enthalten ist, welcher der bevorzugten IV-Leitungsarchitektur zugeordnet ist, Anpassen eines Gewichts, das verwendet wird, um zu bestimmen, ob das Paar medizinischer Vorrichtungen miteinander verbunden ist.

21. Verfahren nach Anspruch 16, wobei das Bestimmen der Paare medizinischer Vorrichtungen, die miteinander verbunden sind, ferner umfasst:
Bestimmen, auf der Grundlage des Typs jeder medizinischen Vorrichtung der Vielzahl medizinischer Vorrichtungen, dass keine medizinischen Vorrichtungen der Vielzahl medizinischer Vorrichtungen einer bevorzugten IV-Leitungsarchitektur zugeordnet sind; und
als Reaktion auf das Bestimmen, dass keine medizinischen Vorrichtungen der Vielzahl medizinischer Vorrichtungen der bevorzugten IV-Leitungsarchitektur zugeordnet sind, Auffordern eines Benutzers, ein weiteres Bild zu erfassen.

22. Verfahren nach Anspruch 15, wobei eine erste Gruppe der Vielzahl medizinischer Vorrichtungen einer Vielzahl von Referenzmarken zugeordnet ist, wobei die Vielzahl von Referenzmarken eine Vielzahl von Kennungen, die der ersten Gruppe medizinischer Vorrichtungen zugeordnet sind, sowie Lageinformationen, die der 3D-Position der Vielzahl von Referenzmarken zugeordnet sind, enthält, und wobei das Bestimmen der Positionsinformationen, die der 3D-Position der Vielzahl medizinischer Vorrichtungen relativ zur Bildaufnahmevorrichtung zugeordnet sind, umfasst:
Bestimmen der Vielzahl von Kennungen, die der ersten Gruppe medizinischer Vorrichtungen zugeordnet sind, sowie der Lageinformationen, die der 3D-Position der Vielzahl von Referenzmarken zugeordnet sind, auf der Grundlage des Bildes,
wobei für jede medizinische Vorrichtung der ersten Gruppe medizinischer Vorrichtungen die Positionsinformationen, die der 3D-Position dieser medizinischen Vorrichtung relativ zur Bildaufnahmevorrichtung zugeordnet sind, als die 3D-Position einer dieser medizinischen Vorrichtung zugeordneten Referenzmarke relativ zur Bildaufnahmevorrichtung bestimmt werden.

23. Verfahren nach Anspruch 22, wobei die Vielzahl von Referenzmarken eine Vielzahl von AprilTags umfasst; oder
wobei für jede medizinische Vorrichtung der ersten Gruppe medizinischer Vorrichtungen die 3D-Position der dieser medizinischen Vorrichtung zugeordneten Referenzmarke relativ zur Bildaufnahmevorrichtung die x-, y- und z-Koordinaten der Referenzmarke sowie Richtungsvektoren für die Z-, Y- und X-Achsen der Referenzmarke umfasst; oder
wobei die Vielzahl von Kennungen einer Vielzahl von Typen medizinischer Vorrichtungen zugeordnet ist; oder
wobei die Vielzahl von Kennungen eine Vielzahl von eindeutigen Kennungen umfasst; oder
wobei eine zweite Gruppe medizinischer Vorrichtungen der Vielzahl medizinischer Vorrichtungen nicht einer Referenzmarke zugeordnet ist, und wobei das Bestimmen der Positionsinformationen, die der 3D-Position der Vielzahl medizinischer Vorrichtungen relativ zur Bildaufnahmevorrichtung zugeordnet sind, ferner umfasst:
für jede medizinische Vorrichtung der zweiten Gruppe medizinischer Vorrichtungen, Bestimmen eines Typs dieser medizinischen Vorrichtung und der 3D-Position dieser medizinischen Vorrichtung relativ zur Bildaufnahmevorrichtung auf der Grundlage des Bildes unter Verwendung einer Objekterkennungstechnik; oder
wobei die Vielzahl von Referenzmarken fest an starren Abschnitten der ersten Gruppe medizinischer Vorrichtungen befestigt ist, sodass sich die Vielzahl von Referenzmarken weder entlang der ersten Gruppe medizinischer Vorrichtungen verschieben noch um diese drehen kann.

24. Verfahren nach Anspruch 15, wobei das Erfassen des Bildes der Vielzahl medizinischer Vorrichtungen ferner umfasst:
Aufnehmen des Bildes der Vielzahl medizinischer Vorrichtungen mit der Bildaufnahmevorrichtung.

25. Verfahren nach Anspruch 24, wobei das Bild eine Bildserie umfasst, wobei die Bildaufnahmevorrichtung die Bildserie vorzugsweise unter Verwendung einer Serienaufnahmetechnik aufnimmt, oder wobei die Bildaufnahmevorrichtung eine Stereokamera aufweist, und wobei die Positionsinformationen, die der 3D-Position der Vielzahl medizinischer Vorrichtungen relativ zur Bildaufnahmevorrichtung zugeordnet sind, unter Verwendung eines Structure-from-Motion-, SfM, Algorithmus bestimmt werden, oder wobei die Bildaufnahmevorrichtung ein LiDAR-System aufweist, und wobei das Bild eine LiDAR-Punktwolke aufweist.

26. Verfahren nach Anspruch 15, wobei das Erzeugen der Darstellung der mindestens einen IV-Leitung das automatische Anzeigen von Linien auf dem Bild umfasst, welche die Paare medizinischer Vorrichtungen verbinden, die als in der mindestens einen IV-Leitung miteinander verbunden bestimmt werden.

27. Verfahren nach Anspruch 15, wobei die mindestens eine IV-Leitung in der Darstellung mindestens einer Pumpe einer Infusionspumpe zugeordnet ist.

28. Verfahren nach Anspruch 15, ferner umfassend:
Bestimmen, mittels des mindestens einen Prozessors, auf der Grundlage der Paare medizinischer Vorrichtungen, bei denen bestimmt wird, dass sie miteinander verbunden sind, einer Verweildauer, die angibt, wie lange eine medizinische Vorrichtung aus der Vielzahl medizinischer Vorrichtungen mit einer anderen medizinischen Vorrichtung aus der Vielzahl medizinischer Vorrichtungen verbunden ist; und
als Reaktion darauf, dass die Verweildauer einen Verweildauerschwellenwert erfüllt, Ausgeben, mittels des mindestens einen Prozessors, einer der medizinischen Vorrichtung zugeordneten Warnmeldung über eine Benutzervorrichtung.

## Revendications

1. Système, comprenant :
au moins un processeur programmé et/ou configuré pour :
obtenir une image d'une pluralité de dispositifs médicaux, capturée par un dispositif de capture d'image ;
déterminer, sur la base de l'image, des informations de position associées à une position tridimensionnelle, 3D, de la pluralité de dispositifs médicaux par rapport au dispositif de capture d'image ;
déterminer, sur la base des informations de position, des paires de dispositifs médicaux de la pluralité de dispositifs médicaux qui sont connectés entre eux ; et
générer, sur la base des paires de dispositifs médicaux qui sont déterminés pour être connectés entre eux, une représentation d'au moins une voie intraveineuse, IV, incluant les paires de dispositifs médicaux qui sont déterminés pour être connectés entre eux.

2. Système selon la revendication 1, dans lequel l'au moins un processeur est programmé et/ou configuré pour déterminer les paires de dispositifs médicaux qui sont connectés entre eux par :
pour chaque paire de dispositifs médicaux de la pluralité de dispositifs médicaux, la détermination, sur la base des informations de position associées à cette paire de dispositifs médicaux, d'une probabilité que les dispositifs médicaux de cette paire de dispositifs médicaux soient connectés entre eux.

3. Système selon la revendication 2, dans lequel l'au moins un processeur est programmé et/ou configuré pour déterminer les dispositifs médicaux des paires de dispositifs médicaux qui sont connectés entre eux par :
pour chaque paire de dispositifs médicaux de la pluralité de dispositifs médicaux, la détermination, sur la base des informations de position associées à cette paire de dispositifs médicaux, des paramètres suivants : une distance entre des points centraux associés à cette paire de dispositifs médicaux, une différence angulaire entre des orientations de cette paire de dispositifs médicaux, et un angle par rapport à la colinéarité de cette paire de dispositifs médicaux, dans lequel la probabilité que cette paire de dispositifs médicaux soit connectée est déterminée sur la base de la distance entre les points centraux de cette paire de dispositifs médicaux, la différence angulaire entre les orientations de cette paire de dispositifs médicaux, et l'angle de colinéarité de cette paire de dispositifs médicaux.

4. Système selon la revendication 2, dans lequel, pour chaque dispositif médical de la pluralité de dispositifs médicaux, ce dispositif médical est déterminé pour être connecté à un autre dispositif médical dans une paire de dispositifs médicaux incluant ce dispositif médical qui est associé à une probabilité la plus élevée des paires de dispositifs médicaux incluant ce dispositif médical.

5. Système selon la revendication 2, dans lequel l'au moins un processeur est programmé et/ou configuré pour déterminer les informations de position associées à la position 3D de la pluralité de dispositifs médicaux par rapport au dispositif de capture d'image par :
la détermination, sur la base de l'image, d'un type de chaque dispositif médical de la pluralité de dispositifs médicaux, dans lequel, pour une paire de dispositifs médicaux de la pluralité de dispositifs médicaux, la probabilité que les dispositifs médicaux de cette paire de dispositifs médicaux soient connectés entre eux est en outre déterminée sur la base du type de chaque dispositif médical dans cette paire de dispositifs médicaux.

6. Système selon la revendication 5, dans lequel l'au moins un processeur est programmé et/ou configuré pour déterminer les paires de dispositifs médicaux qui sont connectés entre eux par :
l'identification, sur la base du type de chaque dispositif médical de la pluralité de dispositifs médicaux, d'un ensemble de dispositifs médicaux de la pluralité de dispositifs médicaux qui sont associés à une architecture de voie IV préférée ; et
pour une paire de dispositifs médicaux inclus dans l'ensemble de dispositifs médicaux associés à l'architecture de voie IV préférée, l'ajustement d'un poids utilisé pour déterminer si les dispositifs médicaux de la paire de dispositifs médicaux sont connectés entre eux.

7. Système selon la revendication 5, dans lequel l'au moins un processeur est programmé et/ou configuré pour déterminer les paires de dispositifs médicaux qui sont connectés entre eux par :
la détermination, sur la base du type de chaque dispositif médical de la pluralité de dispositifs médicaux, qu'aucun dispositif médical de la pluralité de dispositifs médicaux n'est associé à une architecture de voie IV préférée ; et
en réponse à la détermination qu'aucun dispositif médical de la pluralité de dispositifs médicaux n'est associé à l'architecture de voie IV préférée, l'invitation d'un utilisateur à obtenir une autre image.

8. Système selon la revendication 1, dans lequel un premier groupe de dispositifs médicaux de la pluralité de dispositifs médicaux est associé à une pluralité de marqueurs de référence, dans lequel la pluralité de marqueurs de référence encapsule une pluralité d'identifiants associés au premier groupe de dispositifs médicaux et des informations de pose associées à la position 3D de la pluralité de marqueurs de référence, et dans lequel l'au moins un processeur est programmé et/ou configuré pour déterminer les informations de position associées à la position 3D de la pluralité de dispositifs médicaux par rapport au dispositif de capture d'image par :
la détermination, sur la base de l'image, de la pluralité d'identifiants associés au premier groupe de dispositifs médicaux et des informations de pose associées à la position 3D de la pluralité de marqueurs de référence,
dans lequel, pour chaque dispositif médical du premier groupe de dispositifs médicaux, les informations de position associées à la position 3D de ce dispositif médical par rapport au dispositif de capture d'image sont déterminées comme la position 3D d'un marqueur de référence associé à ce dispositif médical par rapport au dispositif de capture d'image.

9. Système selon la revendication 8, dans lequel la pluralité de marqueurs de référence inclut une pluralité d'AprilTags; ou
dans lequel, pour chaque dispositif médical du premier groupe de dispositifs médicaux, la position 3D du marqueur de référence associé à ce dispositif médical par rapport au dispositif de capture d'image inclut des coordonnées x, y et z du marqueur de référence et des vecteurs directionnels pour les axes Z, Y et X du marqueur de référence ; ou
dans lequel la pluralité d'identifiants est associée à une pluralité de types de dispositifs médicaux ; ou
dans lequel la pluralité d'identifiants inclut une pluralité d'identifiants uniques ; ou
dans lequel un second groupe de dispositifs médicaux de la pluralité de dispositifs médicaux n'est pas associé à un marqueur de référence, et dans lequel l'au moins un processeur est programmé et/ou configuré pour déterminer les informations de position associées à la position 3D de la pluralité de dispositifs médicaux par rapport au dispositif de capture d'image par :
pour chaque dispositif médical du second groupe de dispositifs médicaux, la détermination, sur la base de l'image, à l'aide d'une technique de reconnaissance d'objet, d'un type de ce dispositif médical et de la position 3D de ce dispositif médical par rapport au dispositif de capture d'image ; ou
dans lequel la pluralité de marqueurs de référence est fixée de façon rigide à des parties rigides du premier groupe de dispositifs médicaux de sorte que la pluralité de marqueurs de référence ne puisse pas se déplacer le long ni tourner autour du premier groupe de dispositifs médicaux.

10. Système selon la revendication 1, dans lequel l'au moins un processeur est programmé et/ou configuré pour obtenir l'image de la pluralité de dispositifs médicaux en outre par :
la capture, avec le dispositif de capture d'image, de l'image de la pluralité de dispositifs médicaux.

11. Système selon la revendication 10, dans lequel l'image inclut une série d'images, dans lequel de préférence le dispositif de capture d'image capture la série d'images en utilisant une technique de capture en rafale, ou dans lequel le dispositif de capture d'image inclut une caméra stéréo, et dans lequel les informations de position associées à la position 3D de la pluralité de dispositifs médicaux par rapport au dispositif de capture d'image sont déterminées à l'aide d'un algorithme de structure à partir d'un mouvement, SfM, ou
dans lequel le dispositif de capture d'image inclut un système LiDAR, et dans lequel l'image inclut un nuage de points LiDAR.

12. Système selon la revendication 1, dans lequel l'au moins un processeur est programmé et/ou configuré pour générer la représentation de l'au moins une voie IV en affichant automatiquement, sur l'image, des lignes connectant les paires de dispositifs médicaux qui sont déterminées pour être connectés entre eux dans l'au moins une voie IV.

13. Système selon la revendication 1, dans lequel l'au moins une voie IV est associée à au moins une pompe d'une pompe à perfusion dans la représentation.

14. Système selon la revendication 1, dans lequel l'au moins un processeur est programmé et/ou configuré pour :
déterminer, sur la base des paires de dispositifs médicaux qui sont déterminés pour être connectés entre eux, un temps de séjour indiquant une durée pendant laquelle un dispositif médical de la pluralité de dispositifs médicaux est connecté à un autre dispositif médical de la pluralité de dispositifs médicaux ; et
en réponse au fait que le temps de séjour satisfait à un temps de séjour seuil, fournir, via un dispositif utilisateur, une alerte associée au dispositif médical.

15. Procédé, comprenant :
l'obtention, avec au moins un processeur, d'une image d'une pluralité de dispositifs médicaux, capturée par un dispositif de capture d'image ;
la détermination, avec l'au moins un processeur, sur la base de l'image, d'informations de position associées à une position tridimensionnelle, 3D, de la pluralité de dispositifs médicaux par rapport au dispositif de capture d'image ;
la détermination, avec l'au moins un processeur, sur la base des informations de position, de paires de dispositifs médicaux de la pluralité de dispositifs médicaux qui sont connectés entre eux ; et
la génération, avec l'au moins un processeur, sur la base des paires de dispositifs médicaux qui sont déterminés pour être connectés entre eux, d'une représentation d'au moins une voie intraveineuse, IV, incluant les paires de dispositifs médicaux qui sont déterminés pour être connectés entre eux.

16. Procédé selon la revendication 15, dans lequel la détermination des paires de dispositifs médicaux qui sont connectés entre eux inclut en outre :
pour chaque paire de dispositifs médicaux de la pluralité de dispositifs médicaux, la détermination, sur la base des informations de position associées à cette paire de dispositifs médicaux, d'une probabilité que les dispositifs médicaux de cette paire de dispositifs médicaux soient connectés entre eux.

17. Procédé selon la revendication 16, dans lequel la détermination des paires de dispositifs médicaux qui sont connectés entre eux inclut en outre :
pour chaque paire de dispositifs médicaux de la pluralité de dispositifs médicaux, la détermination, sur la base des informations de position associées à cette paire de dispositifs médicaux, des paramètres suivants : une distance entre des points centraux associés à cette paire de dispositifs médicaux, une différence angulaire entre des orientations de cette paire de dispositifs médicaux, et un angle par rapport à la colinéarité de cette paire de dispositifs médicaux, dans lequel la probabilité que cette paire de dispositifs médicaux soit connectée est déterminée sur la base de la distance entre les points centraux de cette paire de dispositifs médicaux, la différence angulaire entre les orientations de cette paire de dispositifs médicaux, et l'angle de colinéarité de cette paire de dispositifs médicaux.

18. Procédé selon la revendication 16, dans lequel, pour chaque dispositif médical de la pluralité de dispositifs médicaux, ce dispositif médical est déterminé pour être connecté à un autre dispositif médical dans une paire de dispositifs médicaux incluant ce dispositif médical qui est associé à une probabilité la plus élevée des paires de dispositifs médicaux incluant ce dispositif médical.

19. Procédé selon la revendication 16, dans lequel la détermination des informations de position associées à la position 3D de la pluralité de dispositifs médicaux par rapport au dispositif de capture d'image inclut en outre :
la détermination, sur la base de l'image, d'un type de chaque dispositif médical de la pluralité de dispositifs médicaux, dans lequel, pour une paire de dispositifs médicaux de la pluralité de dispositifs médicaux, la probabilité que les dispositifs médicaux de cette paire de dispositifs médicaux soient connectés entre eux est en outre déterminée sur la base du type de chaque dispositif médical dans cette paire de dispositifs médicaux.

20. Procédé selon la revendication 19, dans lequel la détermination des paires de dispositifs médicaux qui sont connectés entre eux inclut en outre :
l'identification, sur la base du type de chaque dispositif médical de la pluralité de dispositifs médicaux, d'un ensemble de dispositifs médicaux de la pluralité de dispositifs médicaux qui sont associés à une architecture de voie IV préférée ; et
pour une paire de dispositifs médicaux inclus dans l'ensemble de dispositifs médicaux associés à l'architecture de voie IV préférée, l'ajustement d'un poids utilisé pour déterminer si les dispositifs médicaux de la paire de dispositifs médicaux sont connectés entre eux.

21. Procédé selon la revendication 16, dans lequel la détermination des paires de dispositifs médicaux qui sont connectés entre eux inclut en outre :
la détermination, sur la base du type de chaque dispositif médical de la pluralité de dispositifs médicaux, qu'aucun dispositif médical de la pluralité de dispositifs médicaux n'est associé à une architecture de voie IV préférée ; et
en réponse à la détermination qu'aucun dispositif médical de la pluralité de dispositifs médicaux n'est associé à l'architecture de voie IV préférée, l'invitation d'un utilisateur à obtenir une autre image.

22. Procédé selon la revendication 15, dans lequel un premier groupe de dispositifs médicaux de la pluralité de dispositifs médicaux est associé à une pluralité de marqueurs de référence, dans lequel la pluralité de marqueurs de référence encapsule une pluralité d'identifiants associés au premier groupe de dispositifs médicaux et des informations de pose associées à la position 3D de la pluralité de marqueurs de référence, et dans lequel la détermination des informations de position associées à la position 3D de la pluralité de dispositifs médicaux par rapport au dispositif de capture d'image inclut :
la détermination, sur la base de l'image, de la pluralité d'identifiants associés au premier groupe de dispositifs médicaux et des informations de pose associées à la position 3D de la pluralité de marqueurs de référence,
dans lequel, pour chaque dispositif médical du premier groupe de dispositifs médicaux, les informations de position associées à la position 3D de ce dispositif médical par rapport au dispositif de capture d'image sont déterminées comme la position 3D d'un marqueur de référence associé à ce dispositif médical par rapport au dispositif de capture d'image.

23. Procédé selon la revendication 22, dans lequel la pluralité de marqueurs de référence inclut une pluralité d'AprilTags; ou
dans lequel, pour chaque dispositif médical du premier groupe de dispositifs médicaux, la position 3D du marqueur de référence associé à ce dispositif médical par rapport au dispositif de capture d'image inclut des coordonnées x, y et z du marqueur de référence et des vecteurs directionnels pour les axes Z, Y et X du marqueur de référence ; ou
dans lequel la pluralité d'identifiants est associée à une pluralité de types de dispositifs médicaux ; ou
dans lequel la pluralité d'identifiants inclut une pluralité d'identifiants uniques ; ou
dans lequel un second groupe de dispositifs médicaux de la pluralité de dispositifs médicaux n'est pas associé à un marqueur de référence, et dans lequel la détermination des informations de position associées à la position 3D de la pluralité de dispositifs médicaux par rapport au dispositif de capture d'image inclut en outre :
pour chaque dispositif médical du second groupe de dispositifs médicaux, la détermination, sur la base de l'image, à l'aide d'une technique de reconnaissance d'objet, d'un type de ce dispositif médical et de la position 3D de ce dispositif médical par rapport au dispositif de capture d'image ; ou
dans lequel la pluralité de marqueurs de référence est fixée de façon rigide à des parties rigides du premier groupe de dispositifs médicaux de sorte que la pluralité de marqueurs de référence ne puisse pas se déplacer le long ni tourner autour du premier groupe de dispositifs médicaux.

24. Procédé selon la revendication 15, dans lequel l'obtention de l'image de la pluralité de dispositifs médicaux inclut en outre :
la capture, avec le dispositif de capture d'image, de l'image de la pluralité de dispositifs médicaux.

25. Procédé selon la revendication 24, dans lequel l'image inclut une série d'images, dans lequel de préférence le dispositif de capture d'image capture la série d'images en utilisant une technique de capture en rafale, ou dans lequel le dispositif de capture d'image inclut une caméra stéréo, et dans lequel les informations de position associées à la position 3D de la pluralité de dispositifs médicaux par rapport au dispositif de capture d'image sont déterminées à l'aide d'un algorithme de structure à partir d'un mouvement, SfM, ou
dans lequel le dispositif de capture d'image inclut un système LiDAR, et dans lequel l'image inclut un nuage de points LiDAR.

26. Procédé selon la revendication 15, dans lequel la génération de la représentation de l'au moins une voie IV inclut l'affichage automatique, sur l'image, de lignes connectant les paires de dispositifs médicaux qui sont déterminés pour être connectés entre eux dans l'au moins une voie IV.

27. Procédé selon la revendication 15, dans lequel l'au moins une voie IV est associée à au moins une pompe d'une pompe à perfusion dans la représentation.

28. Procédé selon la revendication 15, comprenant en outre :
la détermination, avec l'au moins un processeur, sur la base des paires de dispositifs médicaux qui sont déterminés pour être connectés entre eux, d'un temps de séjour indiquant une durée pendant laquelle un dispositif médical de la pluralité de dispositifs médicaux est connecté à un autre dispositif médical de la pluralité de dispositifs médicaux ; et
en réponse au fait que le temps de séjour satisfait à un temps de séjour seuil, la fourniture, avec l'au moins un processeur, via un dispositif utilisateur, d'une alerte associée au dispositif médical.
